# EUROPEAN PATENT SPECIFICATION

(11) **EP 1 613 679 B1**
(45) Date of publication and mention of the grant of the patent: **21.09.2011**
(21) Application number: 04758101.2
(22) Date of filing: 24.03.2004
(51) Int. Cl.: C08G 18/42, C08G 18/10, A61L 27/46, A61L 27/54

(54) **METHODS OF PERFORMING MEDICAL PROCEDURES WHICH PROMOTE BONE GROWTH, COMPOSITIONS WHICH PROMOTE BONE GROWTH, AND METHODS OF MAKING SUCH COMPOSITIONS**
VERFAHREN ZUR DURCHFÜHRUNG VON KNOCHENWACHSTUMSFÖRDERNDEN MEDIZINISCHEN PROZEDUREN, KNOCHENWACHSTUMSFÖRDERNDE ZUSAMMENSETZUNGEN UND HERSTELLUNGSVERFAHREN DAFÜR
PROCEDES DE REALISATION D'INTERVENTIONS MEDICALES FAVORISANT LA CROISSANCE OSSEUSE, COMPOSITION FAVORISANT LA CROISSANCE OSSEUSE, ET LEURS PROCEDES DE FABRICATION

(30) Priority: 24.03.2003 US 395001
(43) Date of publication of application: 11.01.2006
(73) Proprietor: Doctor's Research Group, Inc., Southbury, CT 06488 (US)
(72) Inventor: DESLAURIERS, Richard, J., Woodbury, CT 06798 (US); POTASH, Robert, T., Seymour, CT 06483 (US); SENDIJAREVIC, Aisa, Troy, MI 48085 (US)
(74) Representative: Lawrence, John
(86) International application number: PCT/US2004/008966
(87) International publication number: WO 2004/085508

(56) References cited:
- EP-A- 0 867 422
- WO-A-96/39201
- DD-A- 268 114
- DD-A- 268 119
- DD-A- 268 130
- DD-A- 295 827
- US-A- 4 256 617
- US-A- 4 598 136
- US-A1- 2002 016 636

## Description

### BACKGROUND OF THE INVENTION

The present invention relates generally to compositions for use in medical procedures. In particular, the present invention is directed towards compositions for use in medical procedures in which the composition promotes bone growth when the composition is positioned in the vicinity of a bone of a mammal.

Human bone includes a solid mineral phase and an organic matrix which is between 90% and 95% type I collagen. The mineral phase includes, *inter alia,* calcium and phosphate.

The mechanical properties of bone are related to its specific type of construction and internal architecture. Although bone may be relatively light, it also may have a relatively high tensile strength. This combination of high strength coupled with relatively low weight results from, *inter alia,* the hollow, tubular shape of bone, the layering of bone tissue, and the internal buttressing within the organic matrix. Bone tissue may supplant membranous or fibrous tissue by a mechanism referred to as "intramembranous ossification." Bone tissue only grows by appositional growth, *e.g*., the deposition of a new organic matrix on the surface of the bone by adjacent surface cells. A damaged bone repairs itself through a multiphase process. Initially, bone repair begins with an inflammatory phase, involving extensive tearing of the membrane surrounding the bone (the periosteum), rupturing of blood vessels and extensive hemorrhaging. Typically, this leads to a secondary inflammatory response of white blood cells (*e.g*., polymorphonuclear leukocytes, macrophages, and mononuclear cells), in an effort to prevent infection. Pluripotential mesenchymal cells from the soft tissue and within the bone marrow give rise to the osteoblast cells that synthesize bone.

Known bone replacement technologies can be divided into three transitional matrix categories. The first category relies on replacing bone with either autogenous, homologous, heterologous, or decalcified bone, followed by remodeling. As referred to herein, the term "remodeling" will be understood to mean the process by which bone is continually built and resorbed within the body. This first category may be problematic, however, because of difficulties inherent in harvesting the replacement bone, as well as the risk of transmitting blood-borne pathogens into the body of the recipient. The second category involves synthetic bone replacement, *e.g*., replacing bone with a bone-like mineral (*e.g.*, crystalline hydroxyapatite or calcium pyrophosphate), followed by remodeling. Synthetic bone replacement may be problematic, however, because the replacement material may have poor tensile strength and may adhere poorly to the surrounding bone. The third category relies on replacing bone with a composition that maintains its chemical and mechanical properties without change or subsequent remodeling (*e.g*., titanium, stainless steel, PMMA); nevertheless, this category does not allow for the growth of new bone.

### SUMMARY OF THE INVENTION

A technical advantage of the present invention is that a composition of the present invention may be positioned in the vicinity of a bone of a mammal, *e.g*., in the vicinity of a damaged portion of the bone, and the composition promotes bone growth. For example, the composition can be applied to an exterior surface of the bone, dispensed in an opening formed within or through the bone, injected into the bone, positioned between two pieces of bone, or the like, without necessitating exposure of the bone, *e.g*., by injecting the composition through the skin using a syringe. The composition also may be molded into an implant, a screw, a plate, a prosthetic member, or the like, which may be inserted in or positioned on the bone. The composition initially may be liquid, and then may cure into a solid. For example, the composition may cure into a solid in an oxygen environment and/or a hydrophilic environment. The composition may be used to reconstruct bone, fuse bones (intravertebroinfusions), reduce or eliminate bone fractures or otherwise damaged bones, and/or regenerate missing bone, *e.g*., generate bone growth that fills a void within a bone. The composition also may be used to make plates, screws, prosthetic joints, or the like, and/or may act as an anchor for a suture inserted in an opening in a bone, preventing the suture from falling out of the opening after insertion. Moreover, the composition may be used as a base of a substrate in order to dilate compressed structures, *e.g*., vertebral disks, intramedullary nails, and in angioplasty type procedures. The newly generated bone has an internal rigid fixation similar to that of bone already present in the body, such that the generated bone is not readily damaged.

According to the present invention, a composition comprises a polyester urethane component and at least one filler material, wherein the composition is adapted to stimulate bone growth when the composition contacts or is positioned in the vicinity of a bone of a mammal.

The invention provides a composition comprising a polyester urethane and at least one filler material, wherein the composition is adapted to stimulate bone growth when the composition contacts or is positioned in the vicinity of a bone of a mammal, and further wherein the composition is a product of a process comprising the steps of: reacting a polyol, an isocyanate or isocyanate prepolymer, and water to produce a polyester urethane and an amount of carbon dioxide to impart porosity to the composition, wherein the polyol is chosen from a naturally occurring polyol, a biocompatible, synthetic polyol or combinations thereof, and wherein the at least one filler material is calcium carbonate.

The composition of the present invention, may be used in a method of performing a medical procedure which comprises at least one step selected from the group consisting of: applying a particular composition to at least one portion of a bone of a mammal, positioning the particular composition in the vicinity of the bone, dispensing the particular composition into an opening formed within or through at least one portion of the bone, and positioning the particular composition between a first bone portion of the mammal and a second bone portion of the mammal for fusing the first bone portion to the second bone portion, wherein the particular composition is as defined above.

The composition of the present invention, may be used in a method of performing a medical procedure which comprises the steps of: forming a mold; dispensing a liquid, particular composition into the mold, wherein the particular composition solidifies within the mold; removing the solidified, particular composition from the mold; and positioning the solidified, particular composition on a bone of a mammal or within an opening formed through or in the bone; wherein the positioned, particular composition is as defined above.

Other objects, features, and advantages will be apparent to persons of ordinary skill in the art in view of the following detailed description of the invention and the accompanying drawings.

### BRIEF DESCRIPTION OF THE DRAWINGS

For a more complete understanding of the present invention, needs satisfied thereby, and objects, features, and advantages thereof, reference now is made to the following descriptions taken in connection with the accompanying drawings.

**Fig. 1** is a top level flow-chart depicting a method for making a composition according to an exemplary embodiment of the present invention.

**Fig. 2** is a top level flow-chart depicting a method for making a composition according to another exemplary embodiment of the present invention.

**Fig. 3** is a top level flow-chart depicting a method for making a composition according to yet another exemplary embodiment of the present invention.

**Fig. 4** is a top level flow-chart depicting a method for making a composition according to still another exemplary embodiment of the present invention.

**Fig. 5** is a top level flow-chart depicting a method for making a composition according to still yet another exemplary embodiment of the present invention.

**Fig. 6** is a top level flow-chart depicting a method for making a composition according to still yet another exemplary embodiment of the present invention.

**Fig. 7** is a top level flow-chart depicting a method for making a composition according to still yet another exemplary embodiment of the present invention.

**Fig. 8** is a top level flow-chart depicting a method for making a composition according to still yet another exemplary embodiment of the present invention.

**Fig. 9** is a top level flow-chart depicting a method for making a composition according to still yet another exemplary embodiment of the present invention.

**Fig. 10** is a top level flow-chart depicting a method for making a composition according to still yet another exemplary embodiment of the present invention.

**Fig. 11** is a top level flow-chart depicting a method for making a composition according to still yet another exemplary embodiment of the present invention.

**Fig. 12** is a top level flow-chart depicting a method for making a composition according to still yet another exemplary embodiment of the present invention.

**Fig. 13** is a top level flow-chart depicting a method for making a composition according to still yet another exemplary embodiment of the present invention.

**Fig. 14** is a top level flow-chart depicting a method for making a composition according to still yet another exemplary embodiment of the present invention.

**Fig. 15** is a top level flow-chart depicting a first method for performing a medical procedure that can be carried out using the composition of the present invention.

**Fig. 16** is a top level flow-chart depicting a second method for performing a medical procedure that can be carried out using the composition of the present invention.

**Fig. 17** is a top level flow-chart depicting a third method for performing a medical procedure that can be carried out using the composition of the present invention.

**Fig. 18** is a top level flow-chart depicting a fourth method for performing a medical procedure that can be carried out using the composition of the present invention.

**Fig. 19** is a top level flow-chart depicting a fifth method for performing a medical procedure that can be carried out using the composition of the present invention.

**Fig. 20** is a top level flow-chart depicting a sixth method for performing a medical procedure that can be carried out using the composition of the present invention.

**Fig. 21** is a top level flow-chart depicting a seventh method for performing a medical procedure that can be carried out using the composition of the present invention.

While the present invention is susceptible to various modifications and alternative forms, specific exemplary embodiments thereof have been shown in the drawings and are herein described.

### DETAILED DESCRIPTION OF PREFERRED EMBODIMENTS

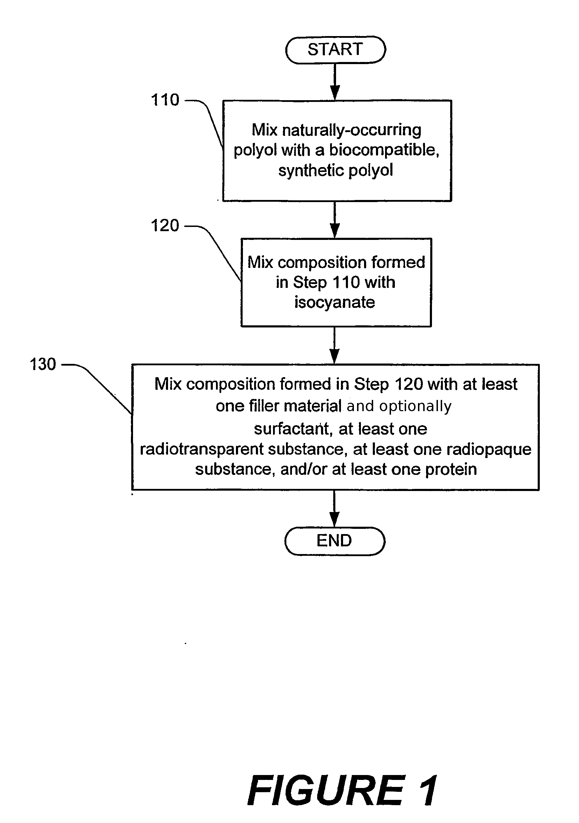

Exemplary embodiments of the present invention and their advantages may be understood by referring to **Figs.** 1-21, like numerals being used for like corresponding parts in the various drawings.

The composition of the present invention is a composition that comprises a biocompatible polyester urethane component and at least one filler material. Generally, the compositions of the present invention that comprise a biocompatible polyester urethane component are those that have been prepared by combining an isocyanate or isocyanate prepolymer with one or more polyols, along with optional additives, and permitting them to react to form a composition that comprises a biocompatible polyester urethane component. The biocompatible polyester urethane component may be present in certain exemplary embodiments of the compositions of the present invention in an amount in the range of from 40% to 90% by weight. In certain exemplary embodiments of the compositions of the present invention, the biocompatible polyester urethane component is present in the composition in an amount in the range of from 50% to 70% by weight. Optionally, the compositions comprising a biocompatible polyester urethane component further may comprise other additives (*e.g*., at least one catalyst, surfactants, proteins, etc.), as will be described.

When placed within the body of a mammal according to the methods as described herein, the compositions of the present invention that comprise a biocompatible polyester urethane component may provide significant advantages over conventional biological materials. For example, other conventional biological materials (*e.g*., those comprising polylactic acid) may degrade over a particular period of time, irrespective of whether new bone has formed in the vicinity of the material. Such degradations may limit the stability of surrounding structures within the body of the mammal. In contrast to these conventional biological materials, the compositions of the present invention are degraded through a cyclic AMP regulated lipase hydrolysis reaction process that generally maintains a 1-to-1 conversion (*e.g*., the compositions of the present invention generally are replaced or converted to bone while maintaining regional structural stability). Additionally, conventional biological materials with osteoconductive properties often have an open cellular matrix to support osteoclasts and osteoblasts in growth. While certain exemplary embodiments of the compositions of the present invention may be considered to have a closed cellular matrix, the compositions of the present invention support osteoclasts in growth through local degradations that create micro-open-cellular matrices. Such micro-open-cellular matrices support osteoclast and osteoblast activity, *e.g*., the osteoclast metabolic activities create an open-cell matrix.

The compositions of the present invention that comprise a biocompatible polyester urethane component generally reside in a moldable state at room temperature for a desired time. Certain exemplary embodiments of the compositions of the present invention that comprise a biocompatible polyester urethane component may reside in a moldable state at room temperature for up to 20 minutes after their formulation; certain other exemplary embodiments may reside in a moldable state at room temperature for a longer or shorter period of time. Absent supplemental heating or cooling, the compositions of the present invention that comprise a biocompatible polyester urethane component generally may solidify at room temperature at a time in the range of from 20 minutes to 30 minutes after their formulation. Where an operator desires to solidify the compositions of the present invention, the time required for solidification may be extended by cooling the composition, or may be reduced by heating the composition. Within 48 hours after their formulation, certain exemplary embodiments of the compositions of the present invention that comprise a biocompatible polyester urethane component may attain a final, cured state; certain other exemplary embodiments of the compositions of the present invention that comprise a biocompatible polyester urethane component may attain a final, cured state at an earlier time. Certain exemplary embodiments of the compositions of the present invention that comprise a biocompatible polyester urethane component may have an average porosity in the range of from 5 to 500 microns; certain other exemplary embodiments may have an average porosity that is less, or that is greater. Certain exemplary embodiments of the compositions of the present invention that comprise a biocompatible polyester urethane component may have an average porosity in the range of from 5 to 100 microns. Certain exemplary embodiments of the compositions of the present invention that comprise a biocompatible polyester urethane component are resistant to thermal degradation up to temperatures of 300 F. Certain exemplary embodiments may be capable of withstanding traditional autoclave sterilization cycles. For example, certain exemplary embodiments of the compositions of the present invention that comprise a biocompatible polyester urethane component repeatedly may be sterilized in an autoclave without substantially affecting their mechanical properties. Certain exemplary embodiments of the compositions of the present invention that comprise a biocompatible polyester urethane component demonstrate a compressive strength of at least 50 MPa, a tensile strength of at least 40 MPa, and/or a Young's Modulus of Elasticity of at least 1,500 MPa. Certain other exemplary embodiments may demonstrate greater or lesser compressive strength, tensile strength, and/or Young's Modulus.

The compositions of the present invention are biocompatible, and are adapted to stimulate bone growth when positioned in contact with, or in the vicinity of, a bone of a mammal. Certain exemplary embodiments of the compositions of the present invention may be adhesive and cohesive, and certain exemplary embodiments may be bacterial static and bactericidal. Certain exemplary embodiments of the compositions of the present invention also may be osteoinductive or osteoconductive. Certain exemplary embodiments of the compositions of the present invention may be suitable for use as a USP Class VI medical adhesive.

### THE ISOCYANATE COMPONENT

A broad variety of isocyanates and isocyanate prepolymers may be suitable for use in the present invention. In certain exemplary embodiments of the present invention, the isocyanate may be, *e.g*., an aromatic isocyanate, an aliphatic isocyanate, a cycloaliphatic isocyanate. An example of a suitable aromatic isocyanate is diphenylmethane isocyanate, also known as "MDI." Commercially available examples of diphenylmethane isocyanate include, but are not limited to, mixtures of 2,4-diphenylmethane isocyanate and 4,4-diphenylmethane isocyanate isomers, such as those that are commercially available from Dow Chemical Company under the tradename ISONATE 50 OP, and those that are commercially available from Huntsman under the tradename RUBINATE 9433; these mixtures of 2,4- and 4,4-diphenylmethane isocyanate isomers generally will be liquids at room temperatures. Diphenylmethane isocyanate is also commercially available, *inter alia,* in its pure 4,4-diphenylmethane isocyanate form from Bayer AG under the tradename MONDUR M, and from Huntsman Corporation under the tradename RUBINATE 44; these compounds generally will be solids at room temperature. Other examples of suitable aromatic isocyanates include, but are not limited to, polymeric isocyanates, such as those that are commercially available from Dow Chemical Company under the tradenames ISONATE 143L, ISONATE PAPI 901, ISONATE PAPI 27. Examples of suitable cycloaliphatic isocyanates include, but are not limited to, isophorone diisocyanate and dicyclohexylmethane diisocyanate. Isophorone diisocyanate is commercially available from Bayer Corporation under the trade name DESMODUR I. Dicyclohexyl methane diisocyanate is commercially available from Bayer Corporation under the trade name DESMODUR W. An example of a suitable aliphatic isocyanate is 1,6 hexylmethylene diisocyanate.

In certain exemplary embodiments of the present invention, the isocyanate may be chosen so that the compositions of the present invention will be liquids at room temperature and will have desired flexural properties. For example, an exemplary embodiment of a composition of the present invention that was prepared from an isocyanate prepolymer that comprised ISONATE PAPI 901 demonstrated flexural strength of 64.9 MPa at 5.5% strain (no yield), while another exemplary embodiment of a composition of the present invention that was prepared from an isocyanate prepolymer that comprised ISONATE 50 OP exhibited flexural strength of 57.4 MPa at 7.0% strain (yield). Furthermore, the quantity of isocyanate that may be used in the present invention will also depend on factors including, *inter alia,* the desired flexural properties of the composition. In certain exemplary embodiments of the present invention a isocyanate prepolymer that comprises an isocyanate is used, and the isocyanate may be generally present in the isocyanate prepolymer in an amount in the range of from 30% to 80% by weight of the isocyanate prepolymer. In certain exemplary embodiments, the isocyanate may be present in the isocyanate prepolymer in an amount in the range of from 30% to 70% by weight of the isocyanate prepolymer. One of ordinary skill in the art, with the benefit of this disclosure, will be able to identify a suitable amount of isocyanate to include in the compositions of the present invention for a particular application.

### THE POLYOL COMPONENT

A broad variety of polyols may be suitable for use in the present invention, specifically, naturally occurring polyols and biocompatible, synthetic polyols, and mixtures thereof. The polyols used in the present invention generally comprise at least one ester group. In certain exemplary embodiments, a polyol used in the present invention may comprise in the range of from 2 to 3 ester groups. In certain exemplary embodiments, a polyol used in the present invention may comprise in the range of from 5 to 10 ester groups.

As referred to herein, the term "naturally occurring polyols" will be understood to include, *inter alia,* naturally occurring polyols as well as polyols that are derived from various vegetable oils. Generally, the naturally occurring polyols that are suitable for use in the present invention are those that have at least one hydroxyl group. In certain exemplary embodiments, the naturally occurring polyols have two or more hydroxyl groups. Examples of naturally occurring polyols include, but are not limited to, castor oil, safflower oil, lesquerella oil, the polyols that may be obtained by chemical modification of naturally occurring vegetable oils (*e.g*., castor oil, olive oil, sesame oil, corn oil), naturally occurring oils that have been trans-esterified (*e.g*., a modified castor oil polyol that has been prepared by the transesterification reaction of natural castor oil with suitable crosslinkers (*e.g*., glycerol, trimethylolpropane)), naturally occurring oils that have been hydrogenated. Another example of a suitable naturally occurring polyol is a difunctional castor-oil-based polyol that is commercially available from CasChem, Inc., under the tradename CASPOL^{®} 5001.

As referred to herein, the term "biocompatible, synthetic polyols" will be understood to include, *inter alia,* biocompatible synthetic polyols that are derived from crude oil. Examples of suitable biocompatible, synthetic polyols include, but are not limited to, polycaprolactone polyols, polyester polyols, polyadipate polyols (*e.g*., poly(hexane-adipate) diol, poly(butane-adipate) diol, poly(ethylene/propylene-adipate) diol, poly(hexane/adipate/isophthalate diol)), polyols that have been derived from a synthetic acid (*e.g*., isophthalic acid, maleic acid). In certain exemplary embodiments, the biocompatible, synthetic polyol may be biodegradable. An example of a suitable polycaprolactone polyol is a polycaprolactone diol that is commercially available from Dow Chemical under the trade name TONE 32 B8.

The The choice of a particular polyol for use in accordance with the present invention may depend on factors including, *inter alia,* the desired flexural properties of the compositions of the present invention that are produced from the particular polyol or polyamine. The use of a relatively short-chain polyol will tend to impart less flexibility to the composition of the present invention than will the use of a relatively long-chain polyol. One of ordinary skill in the art, with the benefit of this disclosure, will be able to identify a suitable polyol for a particular application.

In certain exemplary embodiments of the present invention, the polyol is generally present in the isocyanate prepolymer in an amount in the range of from 10% to 50% by weight of the isocyanate prepolymer. In certain exemplary embodiments, the polyol may be present in the isocyanate prepolymer in an amount in the range of from 20% to 35% by weight of the isocyanate prepolymer. One of ordinary skill in the art, with the benefit of this disclosure, will also be able to identify a suitable amount of polyol to use in the compositions of the present invention for a particular application.

### THE CHAIN-EXTENDER / CROSSLINKER COMPONENT

Examples of suitable crosslinkers include, but are not limited to, trimethylolpropane, glycerine, a trifunctional castor-oil-based polyol that is commercially available from CasChem, Inc., under the tradename CASPOL^{®} 1962, a quadrifunctional castor-oil-based polyol that is commercially available from CasChem, Inc., under the tradename CASPOL^{®} 5004.

Examples of suitable chain-extenders include, but are not limited to, 1,4-butanediol, 1,6-hexanediol, diethylene glycol.

In certain exemplary embodiments wherein a composition of the present invention comprises a chain-extender or crosslinker, the chain-extender or crosslinker may be present in the composition in an amount in the range of 50% to 80% by weight of the isocyanate prepolymer. In certain exemplary embodiments, the chain-extender or crosslinker may be present in the composition in an amount in the range of 60% to 70% by weight of the isocyanate prepolymer.

### WATER

The compositions of the present invention are made by a process in which water is a reactant. The water may be added to the reaction in a variety of ways. For example, formulating the compositions of the present invention in an atmosphere that contains moisture may cause water to become incorporated. As another example, an operator may add a desired amount of water by injecting. Because water is known to react with isocyanate to produce carbon dioxide, the presence of water generates a sufficient amount of carbon dioxide to impart a degree of porosity to the compositions of the present invention. In certain exemplary embodiments of the present invention, water is present in an amount sufficient to provide a desired porosity. In certain exemplary embodiments, water may be present in the compositions of the present invention in an amount in the range of from 0.1% to 1% by weight of the composition. One of ordinary skill in the art, with the benefit of this disclosure, will be able to identify an appropriate amount of water to include in the compositions of the present invention for a particular application.

### THE FILLER MATERIAL COMPONENT

In the compositions at least one filler material is calcium carbonate.

A broad variety of additionel filler materials may be suitable for use in the compositions of the present invention, including, but not limited to, bone (e.g., demineralized bone, allograft bone, and/or autogenous bone), calcium phosphate, calcium pyrophosphate, hydroxyapatite, poly methyl methacrylate, glass-ionomer, calcium sulfate, tricalcium phosphate (e.g., beta tricalcium phosphate), or any combination thereof, or the like. In certain exemplary embodiments, the filler material may be chosen so as to impart a desired degree of porosity in the compositions of the present invention. Generally, the greater the adhesion between the filler material and other components in the composition, the lower the composition's porosity; and vice versa. Generally, the filler material will be present in the compositions of the present invention in an amount sufficient to modify the composition's mechanical properties (*e.g*., Young's Modulus of Elasticity, flexural strength). In certain exemplary embodiments, the filler material is present in the compositions of the present invention in an amount in the range of from 0.01% to 55% by weight. In certain exemplary embodiments, the filler material may be present in the compositions of the present invention in an amount in the range of from 25% to 35% by weight. In certain embodiments, the filler material comprises calcium carbonate in an amount sufficient to provide free calcium to a body of a mammal and enhance osteoconductivity. In certain exemplary embodiments, the filler material comprises at least 98% pure calcium carbonate by weight of the filler material. In certain exemplary embodiments, the calcium carbonate is implantable grade calcium carbonate. In certain exemplary embodiments, the calcium carbonate has a particle size distribution that is capable of enhancing resorption of calcium within the body of a mammal. In certain exemplary embodiments, the particle size distribution further enhances bone remodeling. An example of a suitable additional filler material is poly ether ether ketone (often referred to as "PEEK"). Another example of a suitable additional filler material is commercially available from Cortek, Inc., of Dedham, Massachusetts, under the trade name "Replace™."

### THE OPTIONAL CATALYST COMPONENT

Optionally, certain exemplary embodiments of the compositions of the present invention further may comprise at least one catalyst. In certain exemplary embodiments of the present invention where a catalyst is used, the catalyst may be used by adding the catalyst to a polyol that may be used in the compositions of the present invention. The inclusion of the catalyst in the present invention may permit an operator to control, *inter alia,* certain polymerization reactions that occur during the formulation of the compositions of the present invention (*e.g*., a polymerization reaction between a polyol and a isocyanate prepolymer that comprises an isocyanate). In certain exemplary embodiments of the present invention, at least one catalyst is present in the the present invention in an amount sufficient to ensure that such polymerization reactions have proceeded to completion before the compositions of the present invention are placed within the body of a mammal. This may ensure, *inter alia,* that the isocyanate that may be present within the compositions of the present invention at the time of their placement within the body of a mammal is not free to react while within the body.

A broad variety of catalysts may be used, including, but not limited to, a tertiary amine, and organometallic compounds such as, for example, stannous octoate, and dibutyl tin dilaurate. In certain exemplary embodiments wherein the catalyst is an organometallic catalyst, the presence of the organometallic catalyst in the compositions of the present invention will not adversely impact the radiotransparency or radiopacity of the composition. An example of a suitable tertiary amine is commercially available from Air Products, Inc., under the trade name DABCO 33LV. An example of a suitable source of dibutyl tin dilaurate is commercially available from Air Products, Inc., under the trade name DABCO T12. A tertiary amine may be preferred in, *inter alia,* certain exemplary embodiments of the present invention wherein a catalyst is to be used during preparation of a composition of the present invention that may be placed within a body of a mammal while in liquid form.

In certain exemplary embodiments of the present invention where a catalyst is added to a polyol that may be used in the present invention, the catalyst may be present in the polyol in an amount in the range of from 0.05% to 0.5% by weight of the polyol. In certain exemplary embodiments, the catalyst may be present in the polyol in an amount in the range of from 0.15% to 0.4% by weight of the polyol. One of ordinary skill in the art, with the benefit of this disclosure, will be able to identify a suitable at least one catalyst, and a suitable amount for inclusion in the compositions of the present invention, for a particular application.

### THE OPTIONAL SURFACTANT COMPONENT

Optionally, certain exemplary embodiments of the compositions of the present invention further may comprise at least one surfactant. The inclusion of the at least one surfactant in the present invention may, *inter alia,* impart a desired degree of porosity to the composition, and may permit an operator to control, *inter alia,* the size and/or the shape of pores within the composition. A broad variety of surfactants may be suitable for inclusion in the compositions of the present invention. Commercially available examples of suitable surfactants include, but are not limited to, DABCO DC 193 and DABCO DC 5241, both of which are commercially available from Air Products, Inc., as well as copolymerizable surfactants with phosphate ester functionality that are available under the tradenames "MAXEMUL 6106" and "MAXEMUL 6112" from Uniqema, and silicone surfactants that are commercially available from Struktol Corporation. One of ordinary skill in the art, with the benefit of this disclosure, will be able to identify an appropriate amount of surfactant to include in the compositions of the present invention for a particular application.

### THE OPTIONAL RADIOTRANSPARENT/RADIOPAQUE COMPONENT

Optionally, the compositions of the present invention also may comprise at least one radiotransparent substance or at least one radiopaque substance. The inclusion of such radiotransparent or radiopaque substances in the present invention may be useful, inter alia, when the composition has been placed in contact with, or in the vicinity of, a bone of a mammal, and a physician subsequently seeks to determine the condition or location of the composition or the bone through the use of, inter alia, X-ray photographs. When an exemplary embodiment of the compositions of the present invention includes a radiotransparent substance (e.g., air, nitrogen gas, carbon dioxide, oxygen gas), the attenuation of the composition in the X-ray decreases. Consequently, a physician more readily may visualize the extent to which the underlying damaged bone has been repaired by the bone growth facilitated by treatment with the compositions of the present invention. Similarly, when an exemplary embodiment of the compositions of the present invention includes a radiopaque substance (e.g., insoluble zirconium oxide, a radioactive tracer, a Barium Sulfate contrast media, a gadolinium contrast media, a watersoluble Iodinated contrast media, an oily Iodinated contrast media, an implantable metal (*e.g*., a chip, flake, comprising a metal such as titanium, cobalt, or chromium)), the attenuation of the composition in the X-ray increases. Consequently, the physician more readily may visualize the adequacy of the coverage of the compositions of the present invention on the damaged bone. Moreover, the at least one radiotransparent substance and/or the at least one radiopaque substance may be non-reactive substances, such that they may be included within the compositions of the present invention at any time during the process of manufacturing the compositions. Generally, the at least one radiotransparent substance and/or the at least one radiopaque substance may be present in the compositions of the present invention in an amount in the range of from 5% to 30% by weight of the composition. In certain exemplary embodiments, the at least one radiotransparent substance and/or the at least one radiopaque substance may be present in the compositions of the present invention in an amount in the range of from 10% to 20% by weight of the composition. Examples of commercially available radiopaque substances include "LIPIODOL," "HYPAQUE," and "OMNIPAQUE."

### OTHER OPTIONAL ADDITIVES

Optionally, the compositions of the present invention further may comprise at least one protein. The at least one protein may be used to control the rate of bone regrowth, *e.g*., the type of protein may be selected, such that the protein increases or decreases the rate of bone growth relative to when the at least one protein is not present in the composition. For example, when a physician wishes to closely monitor the bone growth produced by treatment with the compositions of the present invention, the physician may opt to include within the composition at least one protein that tends to decrease the rate of bone growth relative to when the at least one protein is not present in the composition. Moreover, the at least one protein may be non-reactive, such that the at least one protein may be included in the compositions of the present invention at any time during the manufacture of the composition. Examples of suitable proteins include, but are not limited to, OP1 (commercially available from Stryker Homedica), or any recombinant bone morphogenic protein. Further, at least one steroid-based intracellular messenger may be included in the compositions of the present invention, *inter alia,* to modulate the rate of bone growth.

### METHODS OF USE

**Figures 1 through 14** set forth exemplary embodiments of methods of making certain exemplary embodiments of the compositions of the present invention. Referring now to **Fig. 1**, a first compound is formed in step 110 by mixing a naturally occurring polyol with a biocompatible, synthetic polyol. In step 120, the first compound formed in step 110 may be mixed with an isocyanate. In certain exemplary embodiments, the isocyanate and the first compound both may be liquids at room temperature. In an exemplary embodiment, in step 130, the mixture formed in step 120 further is mixed with at least one filler material, and optionally a surfactant, at least one radio transparent substance, at least one radiopaque substance, and/or at least one protein. The process then proceeds to end. As an alternative, the filler and optional additives added in step 130 may be added earlier, *e.g*., in a step (not shown) between step 110 and step 120, as opposed to after step 120; in such alternative embodiments, the process would proceed to end after step 120, the filler and optional additives already having been added in a step (not shown) between step 110 and step 120.

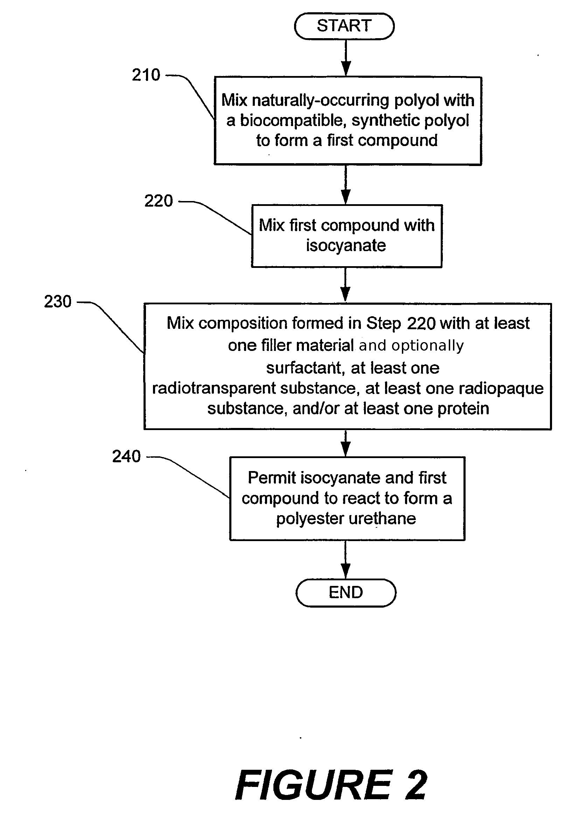

**Figure 2** illustrates an exemplary method for producing a composition of the present invention that comprises a polyester urethane. A first compound is formed in step 210 by mixing a naturally occurring polyol with a biocompatible, synthetic polyol. In step 220, the first compound formed in step 210 may be mixed with an isocyanate. In certain exemplary embodiments, the isocyanate and the first compound both may be liquids at room temperature. In an exemplary embodiment, in step 230, the mixture formed in step 220 is mixed with at least one filler material, and optionally a surfactant, at least one radio transparent substance, at least one radiopaque substance, and/or at least one protein. In step 240, the first compound and the isocyanate are permitted to react to form a polyester urethane. The process then proceeds to end. As an alternative, the filler and optional additives added in step 230 may be added earlier, *e.g*., in a step (not shown) between step 210 and step 220, as opposed to after step 220; in such alternative embodiments, the process would proceed to step 240 after step 220, the filler and optional additives already having been added in a step (not shown) between step 210 and step 220.

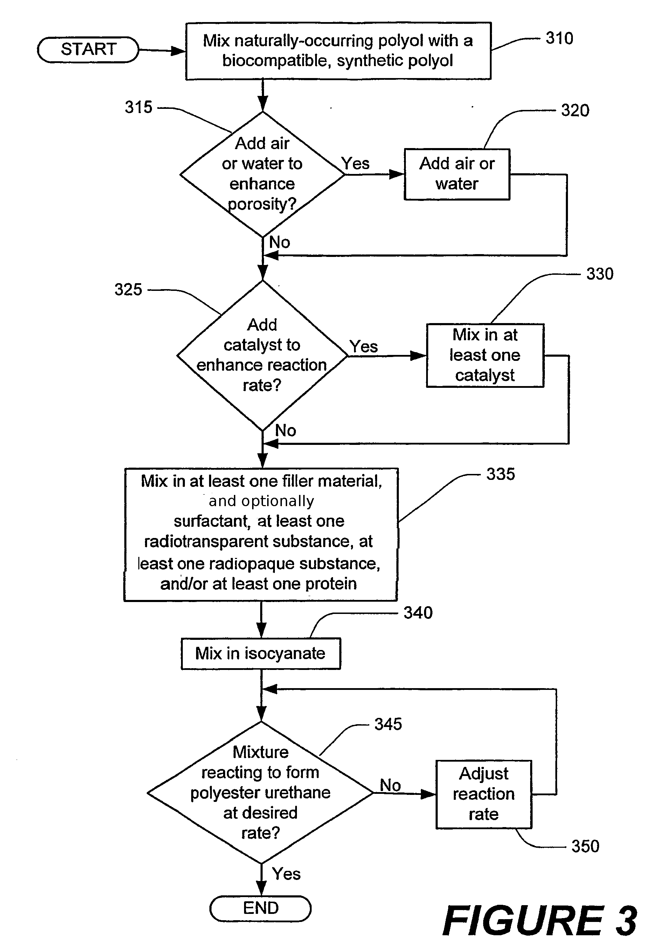

**Figure 3** illustrates an exemplary method for producing a composition of the present invention that comprises a polyester urethane. A first compound is formed in step 310 by mixing a naturally occurring polyol with a biocompatible, synthetic polyol. In step 315, the operator determines whether or not to add air or water to the mixture in an amount that may, *inter alia,* enhance the porosity of the final composition. If the operator elects not to add the air or water, the process proceeds to step 325. If the operator elects to add the optional air or water, the process proceeds to step 320, wherein the operator may add the optional air or water in a variety of ways. For example, the operator may inject water into the mixture in an amount calculated to provide a desired degree of porosity. Alternatively, the operator may inject air into the mixture in an amount calculated to produce a desired degree of porosity. As still another example, the operator may permit air to become entrained into the mixture by pausing mixing for a desired period of time to permit entrainment of a desired amount of air, then resuming mixing after the desired degree of entrainment has occurred. Other means of enhancing the porosity of the mixture are possible, as may be recognized by one of ordinary skill in the art, with the benefit of this disclosure. Optionally, if an operator elects to reduce the porosity of the mixture, the operator may accomplish this, *e.g.,* by vigorously stirring it in a downward motion in such fashion as to force air out of the mixture, thereby reducing the mixture's porosity. From step 320, the process proceeds to step 325, wherein the operator determines whether or not to add an optional catalyst to the mixture; such decision may be based on considerations including, *inter alia,* a desired reaction rate. If the operator elects not to add the optional catalyst, the process proceeds to step 335. If the operator elects to add the optional catalyst, the process proceeds to step 330 wherein the operator may mix in at least one catalyst (*e.g*., a tertiary amine). From step 330, the process proceeds to step 335, wherein the operator mixes in at least one filler material and determines whether to mix in, surfactant, at least one radiotransparent substance, at least one radiopaque substance, and/or at least one protein. From step 335, the process proceeds to step 340, wherein isocyanate is mixed into the mixture, generally over a period of 2 to 3 minutes. In certain exemplary embodiments, the isocyanate and the first compound formed in step 310 both may be liquids at room temperature. From step 340, the process proceeds to step 345, wherein the operator determines whether the first compound and the isocyanate are reacting to form a polyester urethane at a desired rate. If the reaction is not proceeding at a desired rate, the process proceeds to step 350, wherein the operator enhances the reaction rate, *e.g*., by applying or removing heat. From step 350, the process returns to the determination in step 345, which previously has been described. If, however, the operator determines in step 345 that the reaction is proceeding at the desired rate, then the process proceeds to end.

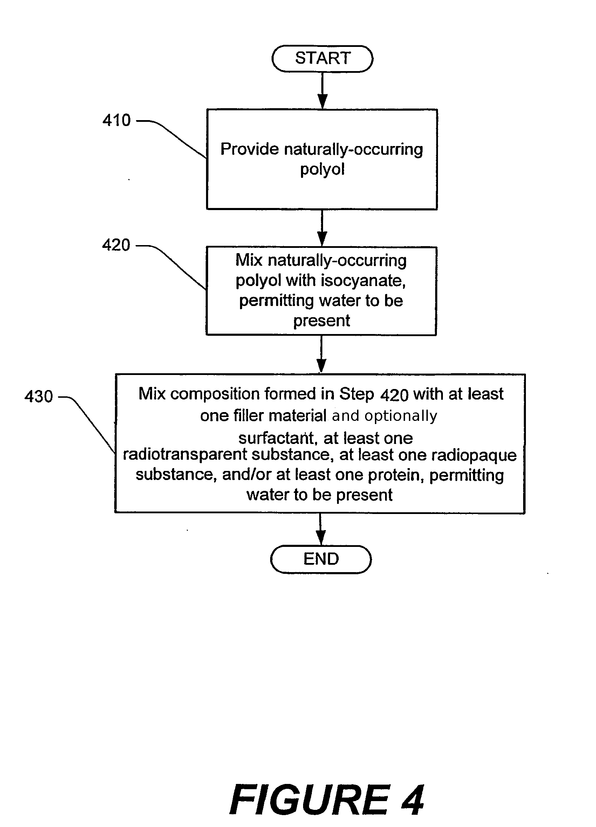

Referring now to **Fig. 4**, a naturally occurring polyol is provided in step 410. In step 420, the naturally occurring polyol may be mixed with an isocyanate, while permitting water to be present. In certain exemplary embodiments, the isocyanate and the naturally occurring polyol both may be liquids at room temperature. In step 430, the mixture formed in step 420 is mixed with at least one filler material, and optionally a surfactant, at least one radio transparent substance, at least one radiopaque substance, and/or at least one protein. The process then proceeds to end. As an alternative, the filler and optional additives added in step 430 may be added earlier, *e.g*., in a step (not shown) between step 410 and step 420, as opposed to after step 420; in such alternative embodiments, the process would proceed to end after step 420, the filler and optional additives already having been added in a step (not shown) between step 410 and step 420.

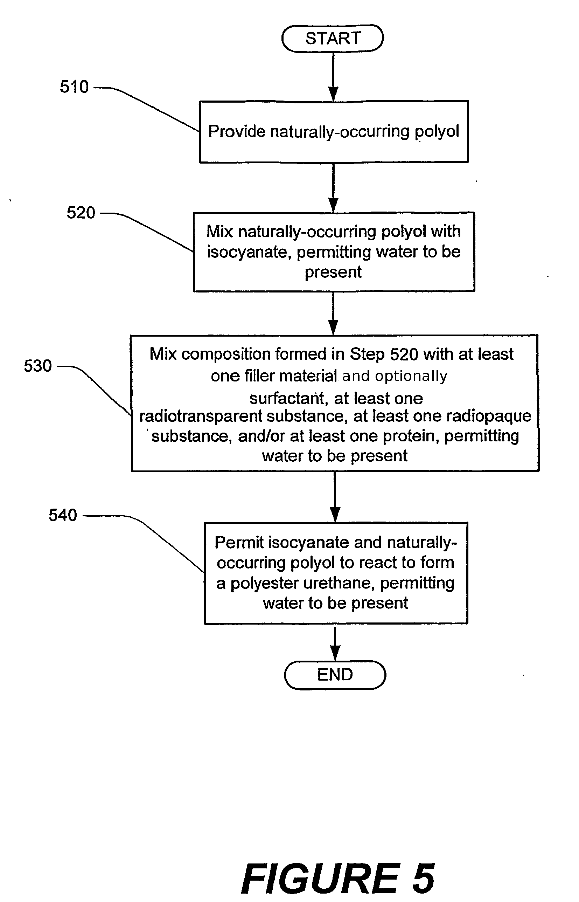

**Figure 5** illustrates an exemplary method for producing a composition of the present invention that comprises a polyester urethane. A naturally occurring polyol is provided in step 510. In step 520, the naturally occurring polyol may be mixed with an isocyanate, while permitting water to be present. In certain exemplary embodiments, the isocyanate and the naturally occurring polyol both may be liquids at room temperature. In step 530, the mixture formed in step 520 is mixed with at least one filler material, and optionally a surfactant, at least one radio transparent substance, at least one radiopaque substance, and/or at least one protein. In step 540, the naturally occurring polyol and the isocyanate are permitted to react to form a polyester urethane, while permitting water to be present. The process then proceeds to end. As an alternative, the filler and optional additives added in step 530 may be added earlier, *e.g*., in a step (not shown) between step 510 and step 520, as opposed to after step 520; in such alternative embodiments, the process would proceed to step 540 after step 520, the filler and optional additives already having been added in a step (not shown) between step 510 and step 520.

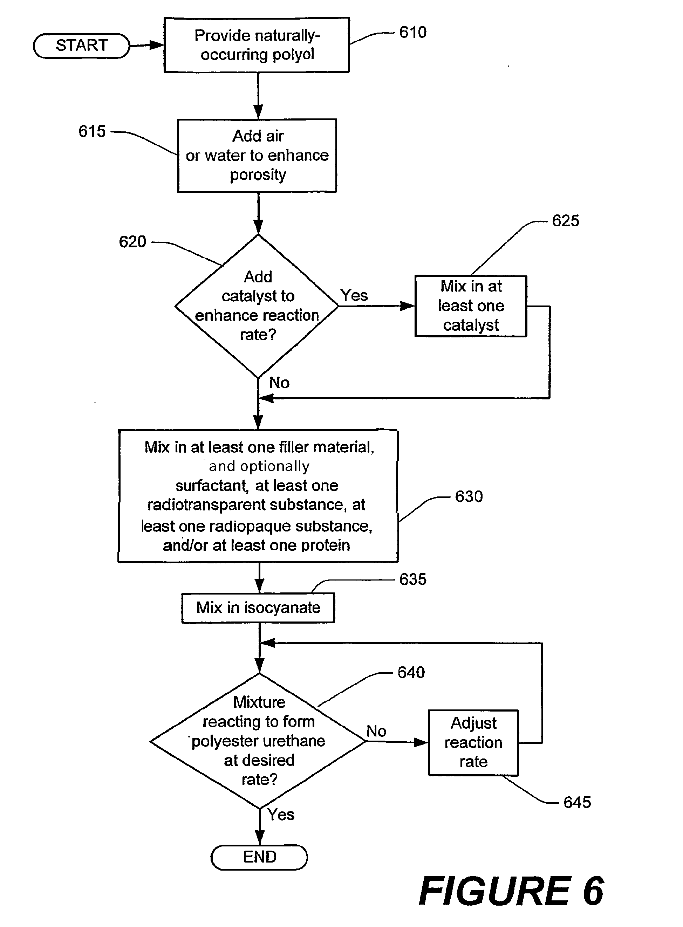

**Figure 6** illustrates an exemplary method for producing a composition of the present invention that comprises a polyester urethane. A naturally occurring polyol is provided in step 610. In step 615, the operator adds water or air to the mixture in an amount that may, *inter alia,* enhance the porosity of the final composition. The operator may add the air or water in a variety of ways. For example, the operator may inject water into the mixture in an amount calculated to provide a desired degree of porosity. Alternatively, the operator may inject air into the mixture in an amount calculated to produce a desired degree of porosity. As still another example, the operator may permit air to become entrained into the mixture by pausing mixing for a desired period of time to permit entrainment of a desired amount of air, then resuming mixing after the desired degree of entrainment has occurred. Other means of enhancing the porosity of the mixture are possible, as may be recognized by one of ordinary skill in the art, with the benefit of this disclosure. Optionally, if an operator elects to reduce the porosity of the mixture, the operator may accomplish this, *e.g*., by vigorously stirring it in a downward motion in such fashion as to force air out of the mixture, thereby reducing the mixture's porosity. From step 615, the process proceeds to step 620, wherein the operator determines whether or not to add an optional catalyst to the mixture; such decision may be based on considerations including, *inter alia,* a desired reaction rate. If the operator elects not to add the optional catalyst, the process proceeds to step 630. If the operator elects to add the optional catalyst, the process proceeds to step 625 wherein the operator may mix in at least one catalyst (*e.g*., a tertiary amine). From step 625, the process proceeds to step 630, wherein the operator mixes in at least one filler material and determines whether to mix in surfactant, at least one radiotransparent substance, at least one radiopaque substance, and/or at least one protein. From step 630, the process proceeds to step 635, wherein isocyanate is mixed into the mixture, generally over a period of about 2 to about 3 minutes. In certain exemplary embodiments, the isocyanate and the naturally occurring polyol both may be liquids at room temperature. From step 635, the process proceeds to step 640, wherein the operator determines whether the naturally occurring polyol and the isocyanate are reacting to form a polyester urethane at a desired rate. If the reaction is not proceeding at a desired rate, the process proceeds to step 645, wherein the operator enhances the reaction rate, *e.g*., by applying or removing heat. From step 645, the process returns to the determination in step 640, which previously has been described. If, however, the operator determines in step 640 that the reaction is proceeding at the desired rate, then the process proceeds to end.

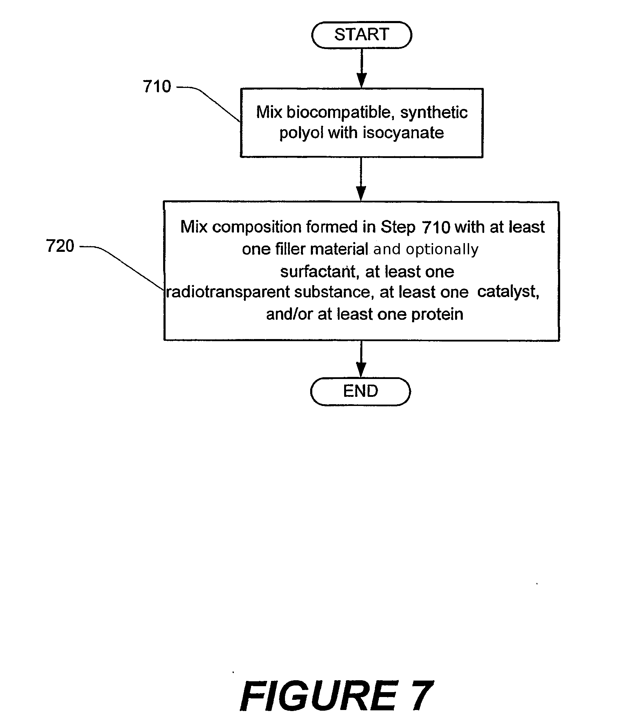

**Figure 7** illustrates another exemplary method of making a composition of the present invention. In step 710, a biocompatible, synthetic polyol may be mixed with an isocyanate. In certain exemplary embodiments of the present invention, the isocyanate and the biocompatible, synthetic polyol both may be liquids at room temperature. In an exemplary embodiment, in step 720, the composition formed in step 710 is mixed with at least one filler material, and optionally a surfactant, at least one radio transparent substance, at least one radiopaque substance, and/or at least one protein. The process then proceeds to end. As an alternative, the filler and optional additives added in step 720 may be added to the biocompatible, synthetic polyol earlier, *e.g*., in a step (not shown) before step 710, as opposed to after step 710; in such alternative embodiments, the process would proceed to end after step 710, the filler and optional additives already having been added in a step (not shown) before step 710.

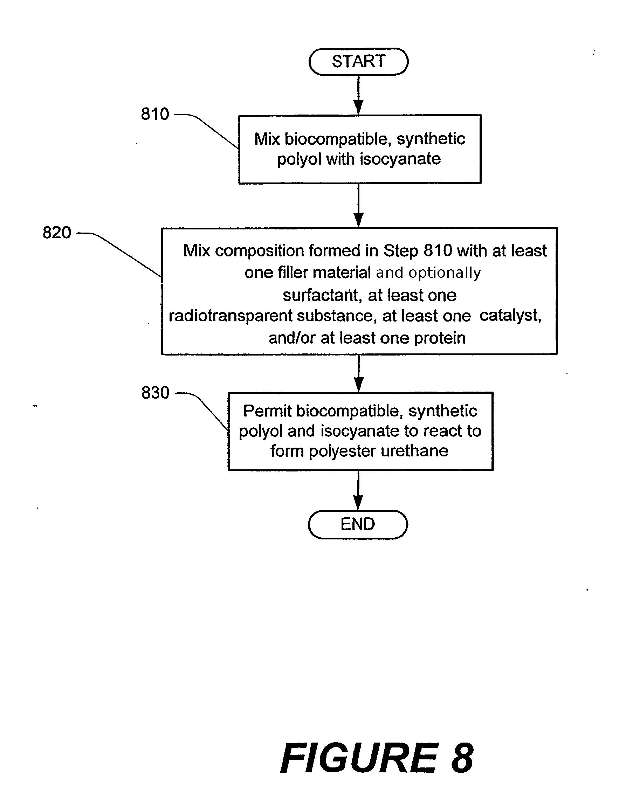

**Figure 8** illustrates an exemplary method of making a composition of the present invention that comprises a polyester urethane component. In step 810, a biocompatible, synthetic polyol may be mixed with an isocyanate. In certain exemplary embodiments, the isocyanate and the biocompatible, synthetic polyol both may be liquids at room temperature. In an exemplary embodiment, in step 820, the composition formed in step 810 is mixed with at least one filler material, and optionally a surfactant, at least one radio transparent substance, at least one radiopaque substance, and/or at least one protein. In step 830, the biocompatible, synthetic polyol and the isocyanate are permitted to react to form a polyester urethane. The process then proceeds to end. As an alternative, the filler and optional additives added in step 820 may be added to the biocompatible, synthetic polyol earlier, *e.g*., in a step (not shown) between before step 810, as opposed to after step 820; in such alternative embodiments, the process would proceed to step 830 after step 810, the fller and optional additives already having been added in a step (not shown) before step 810.

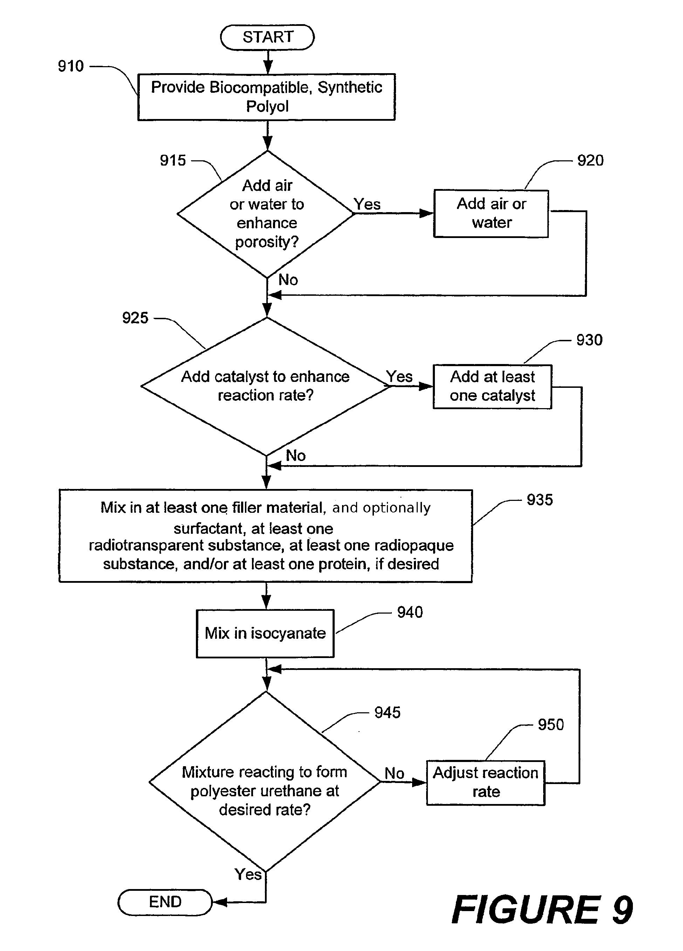

**Figure 9** illustrates another exemplary method of making a composition of the present invention that comprises a polyester urethane component. In step 910, a biocompatible, synthetic polyol is provided. In step 915, the operator determines whether or not to add air or water to the biocompatible, synthetic polyol in an amount that may, *inter alia,* enhance the porosity of the final composition. If the operator elects not to add the air or water, the process proceeds to step 925. If the operator elects to add the optional air or water, the process proceeds to step 920, wherein the operator may add the optional air or water in a variety of ways. For example, the operator may inject water into the mixture in an amount calculated to provide a desired degree of porosity. Alternatively, the operator may inject air into the mixture in an amount calculated to produce a desired degree of porosity. As still another example, the operator may permit air to become entrained into the mixture by pausing mixing for a desired period of time to permit entrainment of a desired amount of air, then resuming mixing after the desired degree of entrainment has occurred. Other means of enhancing the porosity of the mixture are possible, as may be recognized by one of ordinary skill in the art, with the benefit of this disclosure. Optionally, if an operator elects to reduce the porosity of the mixture, the operator may accomplish this, *e.g*., by vigorously stirring it in a downward motion in such fashion as to force air out of the mixture, thereby reducing the mixture's porosity. From step 920, the process proceeds to step 925, wherein the operator determines whether or not to add an optional catalyst to the mixture; such decision may be based on considerations including, *inter alia,* a desired reaction rate. If the operator elects not to add the optional catalyst, the process proceeds to step 935. If the operator elects to add the optional catalyst, the process proceeds to step 930 wherein the operator may mix in at least one catalyst (*e.g*., a tertiary amine). From step 930 the process proceeds to step 935, wherein the operator mixes in at least one filler material and determines whether to mix in surfactant, at least one radiotransparent substance, at least one radiopaque substance, and/or at least one protein. From step 935, the process proceeds to step 940, wherein isocyanate is mixed into the mixture, generally over a period of 2 to 3 minutes. In certain exemplary embodiments, the isocyanate and the biocompatible, synthetic polyol both may be liquids at room temperature. From step 940 the process proceeds to step 945, wherein the operator determines whether the isocyanate and the biocompatible, synthetic polyol are reacting to form a polyester urethane at a desired rate. If the reaction is not proceeding at the desired rate, the process proceeds to step 950, wherein the operator adjusts the reaction rate, *e.g*., by applying or removing heat. From step 950, the process returns to the determination in step 945, which previously has been described. If, however, the operator determines in step 945 that the reaction is proceeding at the desired rate, then the process proceeds to end.

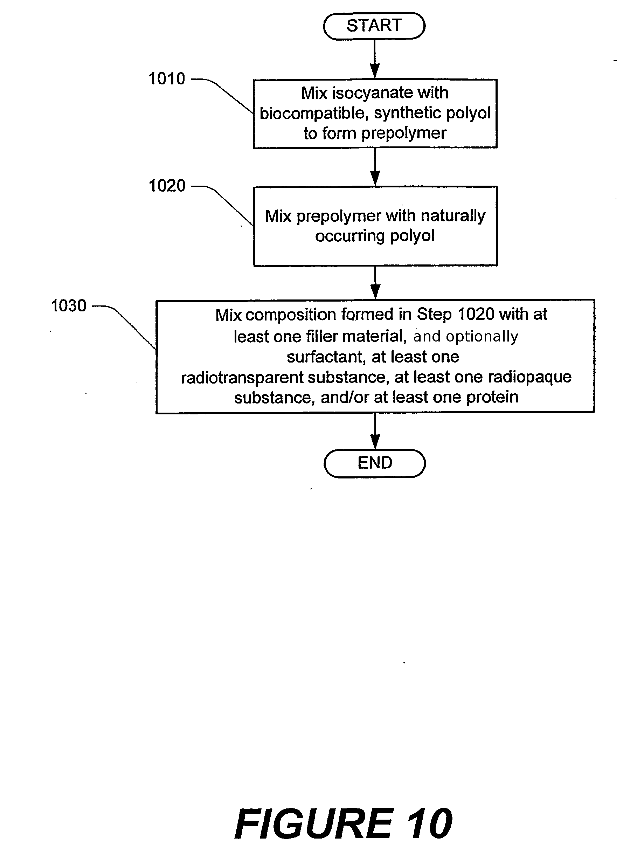

**Figure 10** illustrates still another exemplary method of making a composition of the present invention. In step 1010, a biocompatible, synthetic polyol may be mixed with an isocyanate to form an isocyanate prepolymer. In certain exemplary embodiments of the present invention, the isocyanate may become chemically bound within the isocyanate prepolymer to an extent sufficient to prevent the release of free isocyanate when the composition formed by this method is placed in the body of a mammal. In step 1020, the isocyanate prepolymer formed in step 1010 may be mixed with a naturally occurring polyol. In certain exemplary embodiments, the isocyanate prepolymer and the naturally occurring polyol both may be liquids at room temperature. In an exemplary embodiment, in step 1030, the composition formed in step 1020 is mixed with at least one filler material, and optionally a surfactant, at least one radio transparent substance, at least one radiopaque substance, and/or at least one protein. The process then proceeds to end. As an alternative, the filler and optional additives added in step 1030 may be added earlier, *e.g*., in a step (not shown) between step 1010 and step 1020, as opposed to after step 1020; in such alternative embodiments, the process would proceed to end after step 1020, the filler and optional additives already having been added in a step (not shown) between step 1010 and step 1020.

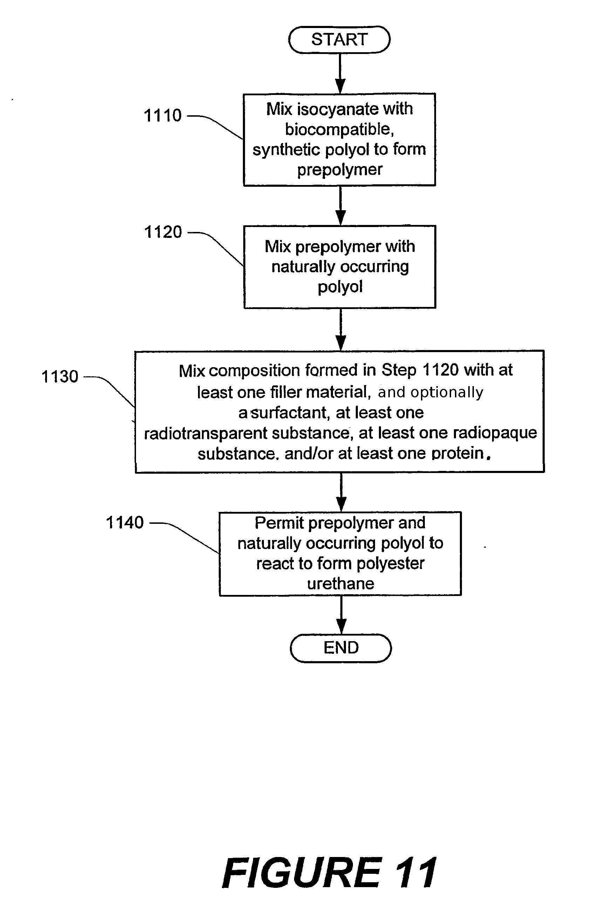

**Figure 11** illustrates still another exemplary method of making a composition of the present invention that comprises a polyester urethane. In step 1110, a biocompatible, synthetic polyol may be mixed with an isocyanate to form an isocyanate prepolymer. In certain exemplary embodiments of the present invention, the isocyanate may become chemically bound within the isocyanate prepolymer to an extent sufficient to prevent the release of free isocyanate when the composition formed by this method is placed in the body of a mammal. In step 1120, the isocyanate prepolymer formed in step 1110 may be mixed with a naturally occurring polyol. In certain exemplary embodiments, the isocyanate prepolymer and the naturally occurring polyol both may be liquids at room temperature. In an exemplary embodiment, in step 1130, the composition formed in step 1120 is mixed with at least one filler material, and optionally surfactant, at least one radio transparent substance, at least one radiopaque substance, and/or at least one protein. In step 1140, the isocyanate prepolymer and the naturally occurring polyol are permitted to react to form a polyester urethane. The process then proceeds to end. As an alternative, the filler and optional additives added in step 1130 may be added earlier, *e.g*., in a step (not shown) between step 1110 and step 1120, as opposed to after step 1120; in such alternative embodiments, the process would proceed to step 1140 after step 1120, the filler and optional additives already having been added in a step (not shown) between step 1110 and step 1120.

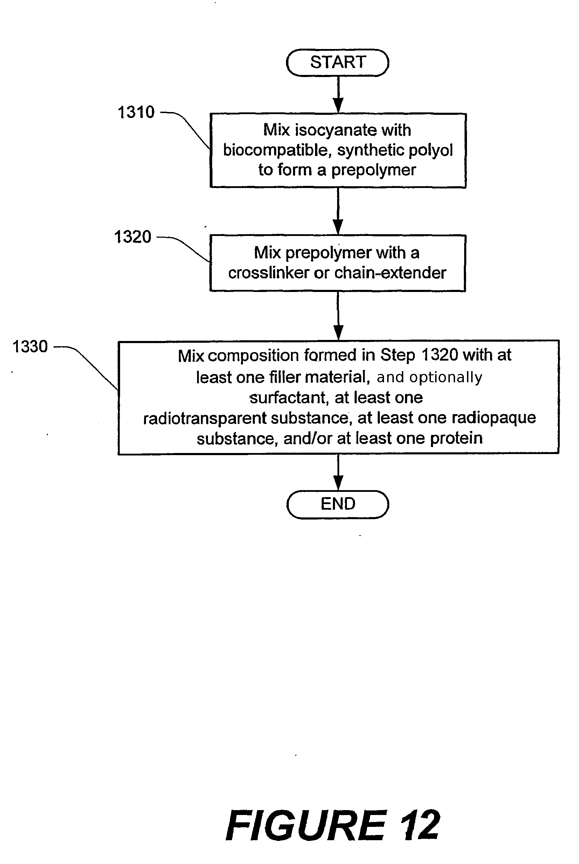

**Figure 12** illustrates another exemplary method of making a composition of the present invention. In step 1310, a biocompatible, synthetic polyol may be mixed with an isocyanate to form an isocyanate prepolymer. In certain exemplary embodiments of the present invention, the isocyanate may become chemically bound within the isocyanate prepolymer to an extent sufficient to prevent the release of free isocyanate when the composition formed by this method is placed in the body of a mammal. In step 1320, the isocyanate prepolymer formed in step 1310 may be mixed with a crosslinker or a chain extender; in certain exemplary embodiments, a crosslinker is used. In certain exemplary embodiments, the isocyanate prepolymer and the crosslinker or chain-extender both may be liquids at room temperature. In an exemplary embodiment, in step 1330, the composition formed in step 1320 is mixed with at least one filler material, and optionaly surfactant, at least one radio transparent substance, at least one radiopaque substance, and/or at least one protein. The process then proceeds to end. As an alternative, the filler and optional additives added in step 1330 may be added earlier, *e.g*., in a step (not shown) between step 1310 and step 1320, as opposed to after step 1320; in such alternative embodiments, the process would proceed to end after step 1320, the filler and optional additives already having been added in a step (not shown) between step 1310 and step 1320.

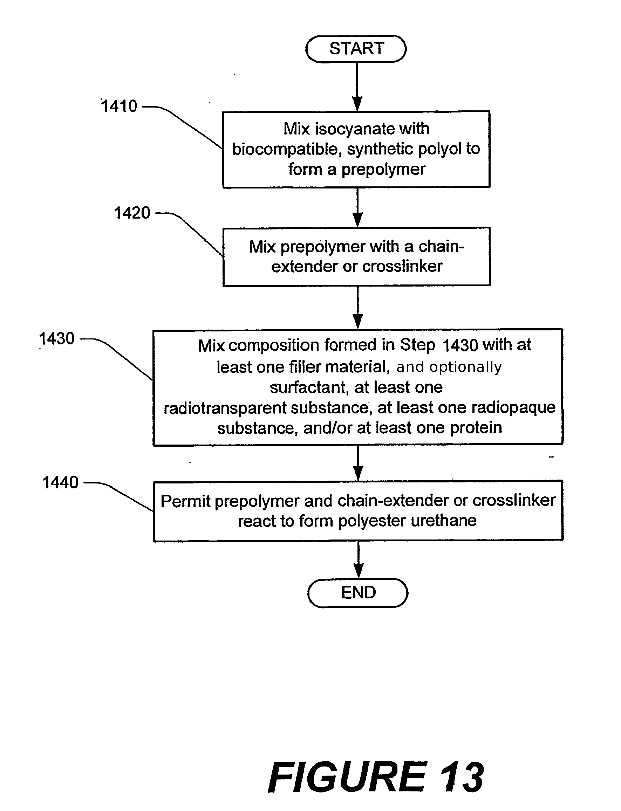

**Figure 13** illustrates another exemplary method of making a composition of the present invention comprising a polyester urethane. In step 1410, a biocompatible, synthetic polyol may be mixed with an isocyanate to form an isocyanate prepolymer. In certain exemplary embodiments of the present invention, the isocyanate may become chemically bound within the isocyanate prepolymer to an extent sufficient to prevent the release of free isocyanate when the composition formed by this method is placed in the body of a mammal. In step 1420, the isocyanate prepolymer formed in step 1410 may be mixed with a crosslinker or chain-extender; in certain exemplary embodiments, a crosslinker is used. In certain exemplary embodiments, the isocyanate prepolymer and the crosslinker both may be liquids at room temperature. In an exemplary embodiment, in step 1430, the composition formed in step 1420 is mixed with at least one filler material, and optionally surfactant, at least one radio transparent substance, at least one radiopaque substance, and/or at least one protein. In step 1440, the isocyanate prepolymer and the vegetable oil are permitted to react to form a polyester urethane. The process then proceeds to end. As an alternative, the filler and optional additives added in step 1430 may be added earlier, *e.g*., in a step (not shown) between step 1410 and step 1420, as opposed to after step 1420; in such alternative embodiments, the process would proceed to step 1440 after step 1420, the filler and optional additives already having been added in a step (not shown) between step 1410 and step 1420.

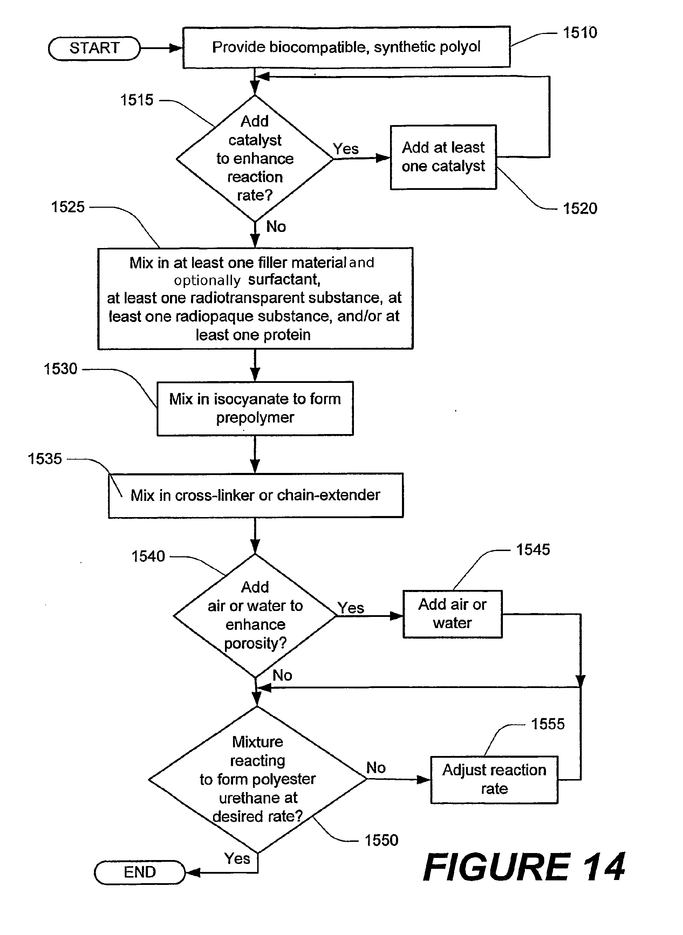

**Figure 14** illustrates still another exemplary method of making a composition of the present invention that comprises a polyester urethane. In step 1510, a biocompatible, synthetic polyol is provided. From step 1510, the process proceeds to step 1515, wherein the operator determines whether or not to add an optional catalyst to the mixture; such decision may be based on considerations including, *inter alia,* a desired reaction rate. If the operator elects not to add the optional catalyst, the process proceeds to step 1525. If the operator elects to add the optional catalyst, the process proceeds to step 1520 wherein the operator may mix in at least one catalyst (*e.g*. a tertiary amine). From step 1520, the process proceeds to step 1525, wherein the mixes in at least one filler material and operator determines whether to mix in surfactant, at least one radiotransparent substance, at least one radiopaque substance, and/or at least one protein. From step 1525, the process proceeds to step 1530, wherein isocyanate is mixed into the mixture, generally over a period of 2 to 3 minutes, so as to form an isocyanate prepolymer. In certain exemplary embodiments, the isocyanate and the biocompatible, synthetic polyol both may be liquids at room temperature. In certain exemplary embodiments of the present invention, the isocyanate may become chemically bound within the isocyanate prepolymer to an extent sufficient to prevent the release of free isocyanate when the composition formed by this method is placed in the body of a mammal. From step 1530, the process proceeds to step 1535, wherein a crosslinker or chain-extender and at least one filler material are mixed into the mixture; in certain exemplary embodiments, a crosslinker is used. In certain exemplary embodiments, the crosslinker or chain-extender and the isocyanate prepolymer both may be liquids at room temperature. In step 1540, the operator determines whether or not to add air or water to the biocompatible, synthetic polyol in an amount that may, *inter alia,* enhance the porosity of the final composition. If the operator elects not to add the air or water, the process proceeds to step 1550. If the operator elects to add the optional air or water, the process proceeds to step 1545, wherein the operator may add the optional air or water in a variety of ways. For example, the operator may inject water into the mixture in an amount calculated to provide a desired degree of porosity. Alternatively, the operator may inject air into the mixture in an amount calculated to produce a desired degree of porosity. As still another example, the operator may permit air to become entrained into the mixture by pausing mixing for a desired period of time to permit entrainment of a desired amount of air, then resuming mixing after the desired degree of entrainment has occurred. Other means of enhancing the porosity of the mixture are possible, as may be recognized by one of ordinary skill in the art, with the benefit of this disclosure. Optionally, if an operator elects to reduce the porosity of the mixture, the operator may accomplish this, *e.g*., by vigorously stirring it in a downward motion in such fashion as to force air out of the mixture, thereby reducing the mixture's porosity. From step 1545, the process proceeds to step 1550, wherein the operator determines whether the isocyanate prepolymer and the crosslinker or chain-extender are reacting to form a polyester urethane at a desired rate. If the mixture is not reacting at a desired rate, the process proceeds to step 1555, wherein the operator enhances the reaction rate, *e.g*., by applying or removing heat. From step 1555, the process returns to the determination in step 1550, which previously has been previously described. If, however, the operator determines in step 1550 that the reaction is proceeding at the desired rate, then the process proceeds to end.

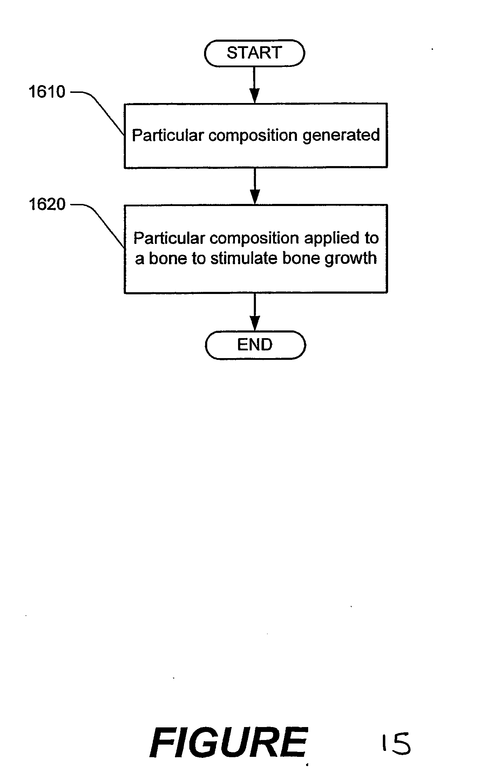

**Figure 15** depicts an exemplary embodiment of a method 1600 for performing a medical procedure, *e.g*., a non-invasive or an invasive medical procedure. In step 1610, a composition of the present invention is generated. The composition is therefore a composition that comprises a polyester urethane. In step 1620, the composition of the present invention may be applied to a portion of a bone of a mammal. For example, a needle may be inserted through a skin of the mammal, and the composition may be dispensed onto a surface of the bone, *e.g*., at a location of a damaged portion of the bone, and the particular composition may stimulate bone growth.

While not willing to be bound by a theory, it is believed that cyclic adenosine monophosphate (cyclic AMP) regulated lipases within the body of a mammal may facilitate the metabolism of the compositions of the present invention after placement of these compositions in contact with, or in the vicinity of, a bone of the mammal. The compositions of the present invention comprise a polyester urethane and are intended to be placed in contact with, or in the vicinity of, a bone of a mammal and generally comprise at least one ester group (*e.g*., a glyceride group) within their chemical structure. Water that naturally is present within the mammal then may react with the at least one ester group so as to be converted into glycerol, fatty acids, and the conversion of adenosine diphosphate to adenosine triphosphate. While not willing to be bound by theory, it is believed that adenosine triphosphate units within the mammal may support various anabolic activities that may result in the formation of bone.

When a composition of the present invention is placed in contact with, or in the vicinity of, a bone of a mammal, the composition may be a liquid, and may conform to a shape of the bone. The composition may transform into a solid after such placement within the mammal. Method 1600 also may comprise the step of increasing or decreasing a temperature of the composition before or after the placement of the composition within the body of the mammal. Increasing the temperature of a composition of the present invention may decrease an amount of time which it takes for the particular composition to transform or cure from a liquid to a solid. Analogously, decreasing the temperature of a composition of the present invention may increase an amount of time which it takes for such transformation or curing of the composition to occur.

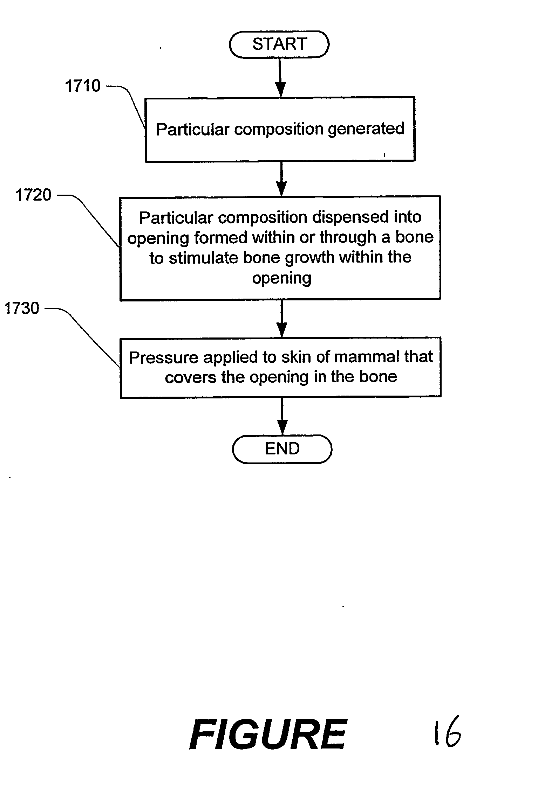

**Figures 16 through 21** illustrate additional exemplary methods of using the composition of the present invention for performing a medical procedure Because certain features and advantages of these exemplary embodiments of these methods are substantially similar to certain features and advantages of the above-described method, such similar features and advantages of the above-described method are not discussed further with respect to the exemplary embodiments of the methods illustrated in **Figures 16 through 21**.

Referring now to **Figure 16****,** another exemplary embodiment of a method 1700 for performing a medical procedure, *e.g*., a non-invasive or an invasive medical procedure, is depicted therein. In step 1710, a composition of the present invention is generated.
In step 1720, the composition is dispensed into an opening formed within or through at least one portion of a bone of a mammal. In an exemplary embodiment, method 1700 also may comprise step 1730, wherein pressure is applied to a skin of the mammal that covers the opening in the bone, which may alter the shape of the composition of the present invention within the opening.

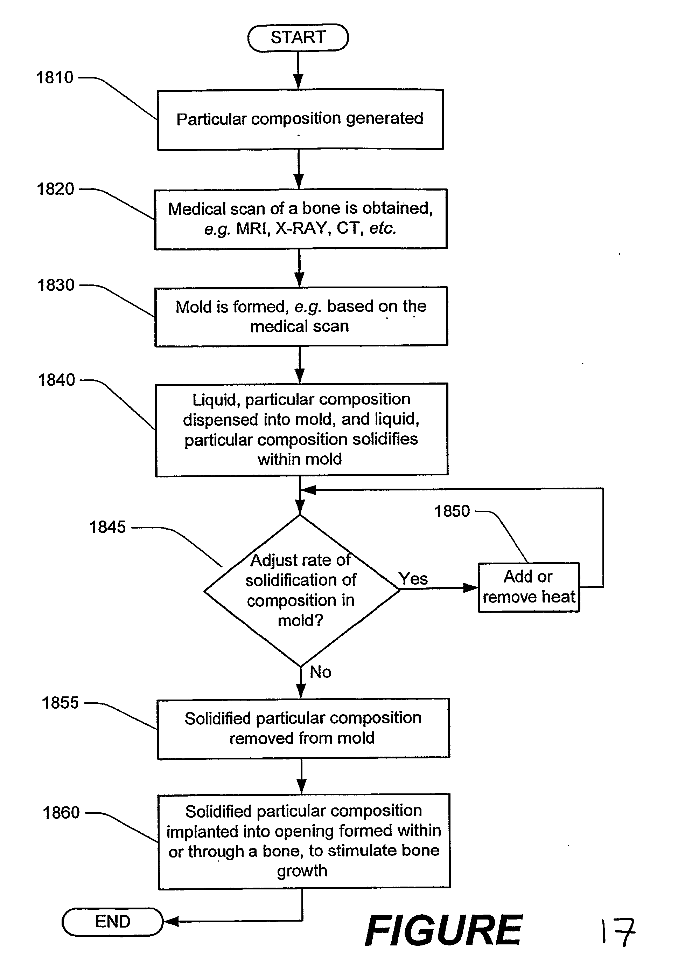

**Figure 17** illustrates still another exemplary embodiment of a method 1800 for performing a medical procedure, *e.g*., a non-invasive or an invasive medical procedure. In step 1810, a composition of the present invention is generated. In step 1820, a medical scan of a bone of a mammal may be obtained, *e.g*., a CT scan, an MRI scan, an X-ray scan, or the like. In step 1830, a mold may be formed, *e.g*., based on the medical scan or based on a generic size for the mold, and in step 1840, the liquid composition of the present invention may be dispensed into the mold, and permitted to solidify therein. In step 1845, the operator determines whether or not to adjust the rate at which the composition solidifies within the mold. If the operator elects to adjust the rate of solidification, the process proceeds to step 1850, wherein the operator adjusts the rate of solidification, *e.g*., by adding or removing heat. Generally, the addition of heat will increase the rate of solidification, whereas the removal of heat will decrease the rate of solidification. From step 1850, the process returns to the determination in step 1845. If, in step 1845, the operator elects not to adjust the rate of solidification, the process proceeds to step 1855. In step 1855, the solidified composition may be removed from the mold, and in step 1860, the solidified composition may be implanted into an opening formed within or through at least one portion of the bone.

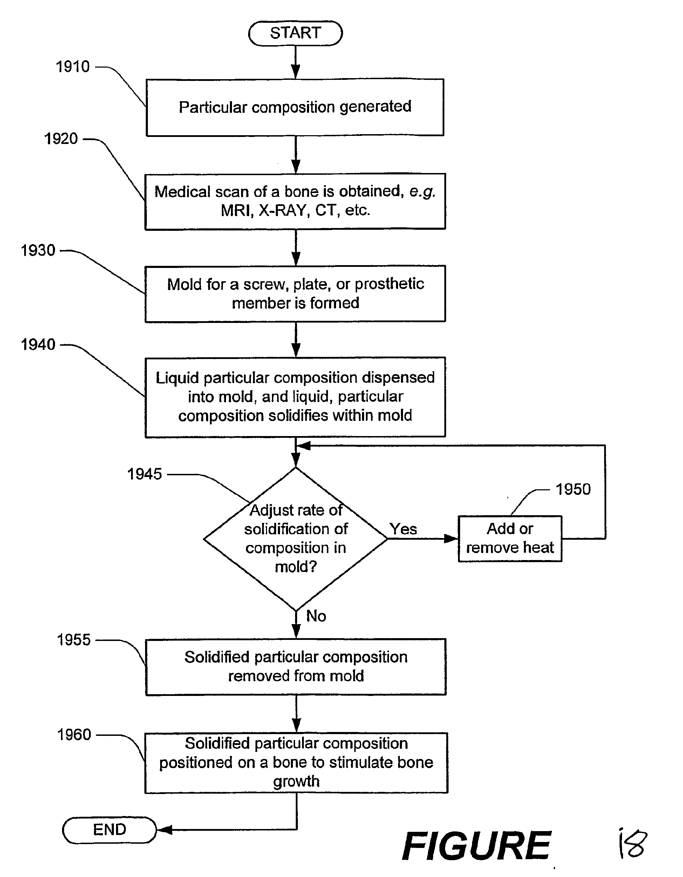

**Figure 18** illustrates another exemplary embodiment of a method 1900 for performing a medical procedure, *e.g*., a non-invasive or an invasive medical procedure. In step 1910, a composition of the present invention is generated. In step 1920, a medical scan of a bone of a mammal may be obtained, *e.g*., a CT scan, an MRI scan, an X-ray scan, or the like. In step 1930, a mold may be formed, *e.g*., based on the medical scan or based on a generic size for the mold, and in step 1940, the liquid composition may be dispensed into the mold, and permitted to solidify therein. In step 1945, the operator determines whether or not to adjust the rate at which the composition solidifies within the mold. If the operator elects to adjust the rate of solidification, the process proceeds to step 1950, wherein the operator adjusts the rate of solidification, *e.g*., by adding or removing heat. Generally, the addition of heat will increase the rate of solidification, whereas the removal of heat will decrease the rate of solidification. From step 1950, the process returns to the determination in step 1945. If, in step 1945, the operator elects not to adjust the rate of solidification, the process proceeds to step 1955. In step 1955, the solidified composition of the present invention may be removed from the mold, and in step 1960, the solidified composition of the present invention may be positioned on at least one portion of the bone.

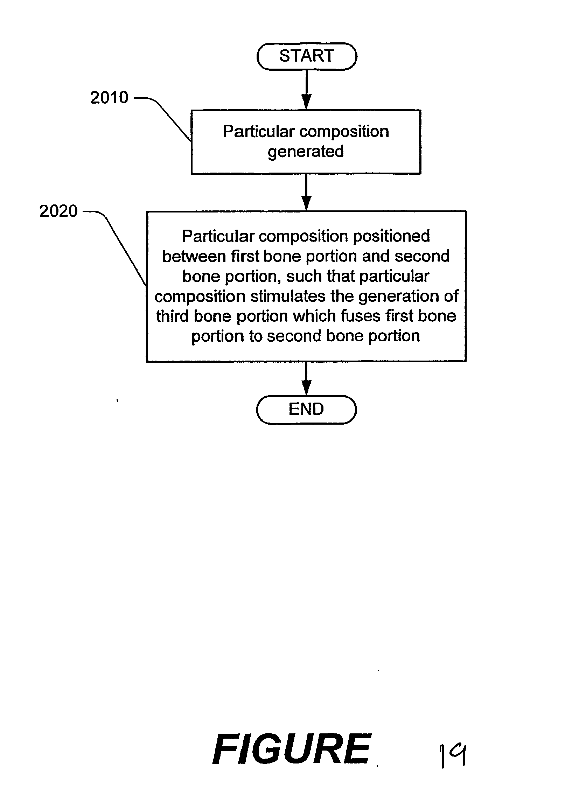

**Figure 19** illustrates still another exemplary embodiment of a method 2000 for performing a medical procedure, *e.g*., a non-invasive or an invasive medical procedure. In step 2010, a particular composition is generated. In step 2020, the composition may be positioned between a first bone portion of a mammal and a second bone portion of the mammal for fusing the first bone portion to the second bone portion, such that the composition stimulates the growth of a third bone portion that fuses the first bone portion to the second bone portion. For example, the composition may be injected into a balloon, and the balloon may be positioned between the first bone portion and the second bone portion. In an exemplary embodiment, the balloon may rest on tissue of the mammal, and the tissue may degrade the balloon before the composition solidifies. Moreover, the same bone within the mammal may comprise each of the first bone portion and the second bone portion, or a first bone may comprise the first bone portion and a second bone may comprise the second bone portion. For example, the first bone may be a first vertebra of a spine of the mammal and the second bone may be a second vertebra of the spine.

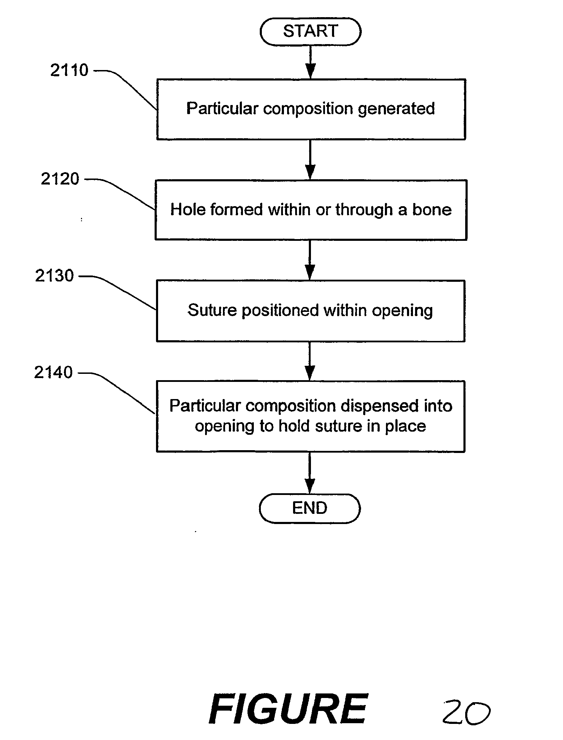

**Figure 20** depicts another exemplary embodiment of a method 2100 for performing a medical procedure, *e.g*., a non-invasive or an invasive medical procedure. In step 2110, a composition is generated. In step 2120, a hole in a bone of a mammal may be formed, *e.g*., drilled, within or through the bone. In step 2130, at least one suture may be positioned within the opening formed within or through the bone. For example, a fluid or a powder which prevents the suture from adhering to the composition of the present invention may be applied to the suture, and then the suture may be dispensed in the opening. In step 2140, the composition of the present invention may be dispensed into the opening to prevent the suture from falling out of the opening.

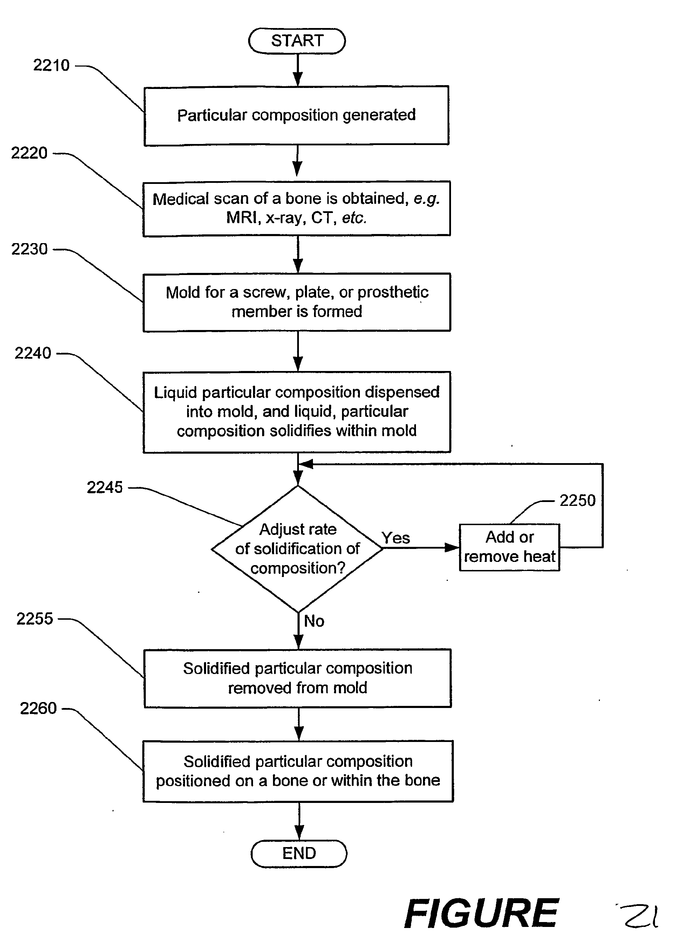

**Figure 21** depicts another exemplary embodiment of a method 2200 for performing a medical procedure, *e.g*., a non-invasive or an invasive medical procedure. In step 2210, a composition is generated.
In step 2220, a medical scan of a bone of a mammal may be obtained, *e.g*., a CT scan, an MRI scan, an X-ray scan, or the like. In step 2230, a mold may be formed, which mold may comprise a mold for a screw, a mold for a plate, a mold for a prosthetic member, or the like. In step 2240, the liquid composition may be dispensed into the mold, and permitted to solidify therein. In step 2245, the operator determines whether or not to adjust the rate at which the composition solidifies within the mold. If the operator elects to adjust the rate of solidification, the process proceeds to step 2250, wherein the operator adjusts the rate of solidification, *e.g*., by adding or removing heat. Generally, the addition of heat will increase the rate of solidification, whereas the removal of heat will decrease the rate of solidification. From step 2250, the process returns to the determination in step 2245. If, in step 2245, the operator elects not to adjust the rate of solidification, the process proceeds to step 2255. In step 2255, the solidified composition of the present invention may be removed from the mold, and in step 2260, the solidified composition of the present invention may be positioned on a bone of a mammal or within an opening formed within the bone.

To facilitate a better understanding of the present invention, the following examples of some exemplary embodiments are given.

### EXAMPLE 1

A sample composition of the present invention was prepared by mixing RUBINATE 9433 with CASPOL^{®} 5001 in a 4:1 equivalent ratio to form an isocyanate prepolymer. Calcium carbonate then was added to the isocyanate prepolymer in an amount of 72.4 % by weight of the isocyanate prepolymer, and mixed for one minute to form a paste. CASPOL^{®} 1962 (containing DABCO 33LV in the amount of 0.20% by weight of the CASPOL^{®} 1962) then was added to the isocyanate prepolymer and calcium carbonate mixture in the amount of 68.65% by weight of the isocyanate prepolymer, and mixed for 2 minutes in a plastic beaker with a spatula. [0100] To prepare test samples for hardness testing, a portion of the above mixture was poured into a cylindrical cavity (dimensions 1 in² by ½ inch) in a Teflon-coated mold, and visually observed for evidence of gelation. Once gelation was detected, the mold was covered with a Teflon-coated plate and placed into a Carver press under slight pressure. Hardness testing was performed according to ASTM 2240.

To prepare test samples for flexural property testing, a portion of the above mixture was poured at its gel time into a cavity of a rectangular aluminum mold (1.5 mm thick) that was coated with a Teflon sheet. Once gelation was detected, the mold was covered with a Teflon-coated plate, placed into a Carver press, and compression molded at about 20,000 psi. The test samples were cut by a saw. Flexural properties were tested according to ASTM D 790 by using an Instron Tester Model 1122 and Merlin software.

The results of the testing are set forth in the table below.

**TABLE 1**

| **Properties** | **Sample Composition No.1** |
|---|---|
| Shore D Hardness | 72 |
| Flexural Strength | 57.4 MPa |
| Strain at Yield | 5.8 |
| Modulus | 2031 MPa |

The above example demonstrates, *inter alia*, that the compositions of the present invention have mechanical properties that may be suitable for bone cements.

### EXAMPLE 2

Sample Composition No. 2, a sample composition of the present invention, was prepared by mixing ISONATE 50 OP with CASPOL^{®} 5001 in a 4:1 equivalent ratio to form an isocyanate prepolymer. Calcium carbonate then was added to the isocyanate prepolymer in an amount of 72.4 % by weight of the isocyanate prepolymer, and mixed for one minute to form a paste. CASPOL^{®} 1962 (containing DABCO 33LV in the amount of 0.35% by weight of the CASPOL^{®} 1962) then was added to the isocyanate prepolymer in the amount of 68.5% by weight of the isocyanate prepolymer, and mixed for 2 minutes in a plastic beaker with a spatula.

To prepare test samples for hardness testing, a portion of the above mixture was poured into a cylindrical cavity (dimensions 1 in² by ½ inch) in a Teflon-coated mold, and visually observed for evidence of gelation. Once gelation was detected, the mold was covered with a Teflon-coated plate and placed into a Carver press under slight pressure. Hardness testing was performed according to ASTM 2240.

To prepare test samples for flexural property testing, a portion of the above mixture was poured at its gel time into a cavity of a rectangular aluminum mold (1.5 mm thick) that was coated with a Teflon sheet. Once gelation was detected, the mold was covered with a Teflon-coated plate, placed into a Carver press, and compression molded at about 20,000 psi. The test samples were cut by a saw. Flexural properties were tested according to ASTM D 790 by using an Instron Tester Model 1122 and Merlin software.

The results of the testing are set forth in the table below.

**TABLE 2**

| **Properties** | **Sample Composition No. 2** |
|---|---|
| Shore D Hardness | 82 |
| Flexural Strength | 84.9 MPa |
| Strain at Yield | 5.9 |
| Modulus | 3193 MPa |

The above example demonstrates, *inter alia*, that the compositions of the present invention have mechanical properties that may be suitable for bone cements.

## Claims

1. A composition comprising a polyester urethane and at least one filler material, wherein the composition is adapted to stimulate bone growth when the composition contacts or is positioned in the vicinity of a bone of a mammal, and further wherein the composition is a product of a process comprising the steps of: reacting a polyol, an isocyanate or isocyanate prepolymer, and water to produce a polyester urethane and an amount of carbon dioxide to impart porosity to the composition, wherein the polyol is chosen from a naturally occurring polyol, a biocompatible, synthetic polyol or combinations thereof, and wherein the at least one filler material is calcium carbonate.

2. The composition of claim 1, wherein the water is present in an amount ranging from 0.1% to 1% by weight of the composition.

3. The composition of claim 1, wherein the at least one filler material is additionally one or more of bone, calcium phosphate, calcium pyrophosphate, hydroxyapatite, poly methyl methacrylate, glass-ionomer, calcium sulfate, or tricalcium phosphate.

4. The composition of claim 1, wherein the isocyanate is an isocyanate prepolymer produced by reacting a diphenylmethane isocyanate mixture of 2,4 diphenylmethane isocyanate and 4,4-diphenylmethane isocyanate with a naturally occurring, difunctional castor-oil based polyol in a 4:1 equivalent ratio, and the process of producing the polyester urethane composition further comprises mixing calcium carbonate to the isocyanate prepolymer in an amount of 72,4% by weight of the isocyanate prepolymer to form a paste, and adding to the paste in an amount of 68,65% by weight of the isocyanate prepolymer, a quadrifunctional castor-oil based polyol crosslinker containing a DABCO catalyst in an amount of 0.2 by weight percent of the crosslinker.

5. The composition of claim 1, the polyol is present in the isocyanate prepolymer in an amount ranging from 10% to 50% by weight of the isocyanate prepolymer, and the polyol is chosen from castor oil, safflower oil, lesquerella oil, chemically-modified vegetable oils, trans-esterified naturally-occurring oils, hydrogenated naturally-occurring oils, difunctional castor-oil based polyols, polycaprolactone polyols, polyester polyols, polyadipate polyols and polyols derived from a synthetic acid.

6. The composition of claim 1, wherein the isocyanate is an isocyanate prepolymer produced by reacting a diphenylmethane isocyanate mixture of 2,4 diphenylmethane isocyanate and 4,4-diphenylmethane isocyanate with a naturally occurring, difunctional castor-oil based polyol in a 4:1 equivalent ratio, and the process of producing the polyester urethane composition further comprises mixing calcium carbonate to the isocyanate prepolymer in an amount of 72,4% by weight of the isocyanate prepolymer to form a paste, and adding to the paste in an amount of 68,5% by weight of the isocyanate prepolymer, a quadrifunctional castor-oil based polyol crosslinker containing a DABCO catalyst in an amount of 0.35 by weight percent of the crosslinker.

7. The composition of claim 4, wherein the composition has a Shore D Hardness of 72, a flexural strength of 57 MPa, a strain at yield of 5.8, and a modulus of 2031 MPa.

8. The composition of claim 6, wherein the composition has a Shore D Hardness of 82, a flexural strength of 85 MPa, a strain at yield of 5.9, and a modulus of 3193 MPa.

9. The composition of claim 1, wherein the composition in its final, cured state has a compressive strength of at least 50 MPa.

10. The composition of claim 1, wherein the composition in its final cured state has a tensile strength of at least 40 MPa.

11. The composition of claim 1, wherein the composition in its final, cured state has a Modulus of Elasticity of at least 1,500 MPa.

12. The composition of claim 1, wherein the composition in its final cured state has a compressive strength of at least 50 MPa, a tensile strength of at least 40 MPa, and a Modulus of Elasticity of at least 1,500 MPa.

## Patentansprüche

1. Zusammensetzung, die ein Polyesterurethan und zumindest ein Füllmaterial aufweist, wobei die Zusammensetzung geeignet ist, Knochenwachstum zu stimulieren, wenn die Zusammensetzung einen Knochen eines Säugetieres kontaktiert oder in dessen Umgebung positioniert ist, und wobei die Zusammensetzung ferner ein Produkt eines Verfahrens ist, das die Schritte umfasst: Reagieren lassen eines Polyols, eines Isocyanats oder eines Isocyanat-Präpolymers, und Wasser, um ein Polyesterurethan herzustellen, und einer Menge an Kohlendioxid, um der Zusammensetzung Porosität zu verleihen, wobei das Polyol aus einem natürlich vorkommenden Polyol, einem biokompatiblen, synthetischen Polyol oder Kombinationen davon ausgewählt ist und wobei das zumindest eine Füllmaterial Kalziumkarbonat ist.

2. Zusammensetzung nach Anspruch 1, wobei das Wasser in einer Menge im Bereich von 0,1 Gew.-% bis 1 Gew.-% der Zusammensetzung vorliegt.

3. Zusammensetzung nach Anspruch 1, wobei das zumindest eine Füllmaterial zusätzlich eines oder mehrere aus Knochen, Kalziumphosphat, Kalziumpyrophosphat, Hydroxyapatit, Polymethyl-Methacrylat, Glas-Ionomer, Kalziumsulfat oder Trikalziumphosphat ist.

4. Zusammensetzung nach Anspruch 1, wobei das Isocyanat ein Isocyanat-Präpolymer ist, das durch reagieren Lassen einer Diphenylmethan-Isocyanat-Mischung aus
2,4-Diphenylmethan-Isocyanat und 4,4-Diphenylmethan-Isocyanat mit einem natürlich vorkommenden, di-funktionellen Polyol auf Rhizinus-Öl-Basis in einem 4:1 Äquivalentverhältnis hergestellt wird, und wobei das Verfahren zum Herstellen der Polyesterurethan-Zusammensetzung ferner das Mischen von Kalziumkarbonat zu dem Isocyanat-Präpolymer in einer Menge von 72,4 Gew.-% des Isocyanat-Präpolymers aufweist, um eine Paste zu bilden, und das Hinzufügen eines quadrifunktionellen Polyol-Vernetzers auf Rhizinus-Öl-Basis, der eine DABCO-Katalysator in einer Menge von 0,2 Gew.-% des Vernetzers enthält, in einer Menge von 68,65 Gew.-% des Isocyanat-Präpolymers zu der Paste.

5. Zusammensetzung nach Anspruch 1, wobei das Polyol in dem Isocyanat-Präpolymer in einer Menge im Bereich von 10 Gew.-% bis 50 Gew.-% des Isocyanat-Präpolymers vorliegt, und das Polyol ausgewählt ist aus Rhizinus-Öl, Distelöl, Lesquerella-Öl, chemisch modifizierten Pflanzenölen, transveresterten, natürlich vorkommenden Ölen, hydrierten, natürlich vorkommenden Ölen, di-funktionellen Polyolen auf Rhizinus-Öl-Basis, Polycaprolaktonpolyolen, Polyesterpolyolen, Polyadipatpolyolen und Polyolen, die von einer synthetischen Säure stammen.

6. Zusammensetzung nach Anspruch 1, wobei das Isocyanat ein Isocyanat-Präpolymer ist, das durch reagieren Lassen einer Diphenylmethan-Isocyanatmischung aus 2,4-Diphenylmethan-Isocyanat und 4,4-Diphenylmethan-Isocyanat mit einem natürlich vorkommenden, di-funktionellen Polyol auf Rhizinus-Öl-Basis in einem 4:1 Äquivalent-Verhältnis hergestellt wird, und das Verfahren zum Herstellen der Polyesterurethan-zusammensetzung ferner das Mischen von Kalziumkarbonat zu dem Isocyanat-Präpolymer in einer Menge von 72,4 Gew.-% des Isocyanat-Präpolymers aufweist, um eine Paste zu bilden, und das Hinzufügen eines quadrifunktionellen Polyol-Vernetzers auf Rhizinus-Öl-Basis, der einen DABCO-Katalysator in einer Menge von 0,35 Gew.-% des Vernetzers enthält, in einer Menge von 68,5 Gew.-% des Isocyanat-Präpolymers zu der Paste.

7. Zusammensetzung nach Anspruch 4, wobei die Zusammensetzung eine Shore-D-Härte von 72, eine Biegefestigkeit von 57 MPa, eine Streckdehnung von 5,8 und ein Modul von 2.031 MPa aufweist.

8. Zusammensetzung nach Anspruch 6, wobei die Zusammensetzung eine Shore-D-Härte von 82, eine Biegefestigkeit von 85 MPa, eine Streckdehnung von 5,9 und ein Modul von 3.193 MPa aufweist.

9. Zusammensetzung nach Anspruch 1, wobei die Zusammensetzung in ihrem endgültigen, ausgehärteten Zustand eine Druckfestigkeit von zumindest 50 MPa aufweist.

10. Zusammensetzung nach Anspruch 1, wobei die Zusammensetzung in ihrem endgültigen, ausgehärteten Zustand eine Bruchfestigkeit von zumindest 40 MPa aufweist.

11. Zusammensetzung nach Anspruch 1, wobei die Zusammensetzung in ihrem endgültigen, ausgehärteten Zustand ein Elastizitätsmodul von zumindest 1.500 MPa aufweist.

12. Zusammensetzung nach Anspruch 1, wobei die Zusammensetzung in ihrem endgültigen, ausgehärteten Zustand eine Druckfestigkeit von zumindest 50 MPa, eine Bruchfestigkeit von zumindest 40 MPa und ein Elastizitätsmodul von zumindest 1.500 MPa aufweist.

## Revendications

1. Composition comprenant un polyester-uréthane et au moins un matériau de charge, laquelle composition est adaptée pour stimuler la croissance de l'os quand la composition vient au contact ou est positionnée au voisinage d'un os d'un mammifère, et en outre laquelle composition est le produit d'un procédé comprenant les étapes consistant à : faire réagir un polyol, un isocyanate ou un prépolymère d'isocyanate, et de l'eau pour produire un polyester-uréthane, et une quantité de dioxyde de carbone pour conférer une porosité à la composition, le polyol étant choisi parmi un polyol naturel, un polyol synthétique biocompatible ou leurs combinaisons, et dans laquelle l'au moins un matériau de charge est le carbonate de calcium.

2. Composition selon la revendication 1, dans laquelle l'eau présente en une quantité située dans la plage allant de 0,1 % à 1 % en poids de la composition.

3. Composition selon la revendication 1, dans laquelle l'au moins un matériau de charge est de plus un ou plusieurs parmi l'os, le phosphate de calcium, le pyrophosphate de calcium, l'hydroxyapatite, le poly(méthacrylate de méthyle), le verre ionomère, le sulfate de calcium, ou le phosphate tricalcique.

4. Composition selon la revendication 1, dans laquelle l'isocyanate est un prépolymère d'isocyanate produit par réaction d'un mélange d'isocyanates de diphénylméthane constitué d'isocyanate de 2,4-diphénylméthane et d'isocyanate de 4,4-diphénylméthane avec un polyol à base d'huile de ricin difonctionnel naturel, en un rapport équivalent de 4/1, et le procédé de production de la composition de polyester-uréthane comprend en outre le mélange de carbonate de calcium au prépolymère d'isocyanate en une quantité de 72,4 % en poids du prépolymère d'isocyanate pour former une pâte, et l'addition à la pâte, en une quantité de 68,65 % en poids du prépolymère d'isocyanate, d'un agent de réticulation de type polyol à base d'huile de ricin tétrafonctionnel contenant un catalyseur DABDO en une quantité de 0,2 % en poids de l'agent de réticulation.

5. Composition selon la revendication 1, dans laquelle le polyol est présent dans le prépolymère d'isocyanate en une quantité située dans la plage allant de 10 % à 50 % en poids du prépolymère d'isocyanate, et le polyol est choisi parmi l'huile de ricin, l'huile de carthame, l'huile de lesquerella, les huiles végétales chimiquement modifiées, les huiles naturelles trans-estérifiées, les huiles naturelles hydrogénées, les polyols à base d'huile de ricin difonctionnels, les polycaprolactone-polyols, les polyester-polyols, les polyadipate-polyols et les polyols dérivés d'un acide synthétique.

6. Composition selon la revendication 1, dans laquelle l'isocyanate est un prépolymère d'isocyanate produit par réaction d'un mélange d'isocyanates de diphénylméthane constitué d'isocyanate de 2,4-diphénylméthane et d'isocyanate de 4,4-diphénylméthane avec un polyol à base d'huile de ricin difonctionnel naturel, en un rapport équivalent de 4/1, et le procédé de production de la composition de polyester-uréthane comprend en outre le mélange de carbonate de calcium au prépolymère d'isocyanate en une quantité de 72,4 % en poids du prépolymère d'isocyanate pour former une pâte, et l'addition à la pâte, en une quantité de 68,5 % en poids du prépolymère d'isocyanate, d'un agent de réticulation de type polyol à base d'huile de ricin tétrafonctionnel contenant un catalyseur DABDO en une quantité de 0,35 % en poids de l'agent de réticulation.

7. Composition selon la revendication 4, laquelle composition a une dureté Shore D de 72, une résistance à la flexion de 57 MPa, une déformation à la limite d'écoulement de 5,8, et un module de 2031 MPa.

8. Composition selon la revendication 6, laquelle composition a une dureté Shore D de 82, une résistance à la flexion de 85 MPa, une déformation à la limite d'écoulement de 5,9, et un module de 3193 MPa.

9. Composition selon la revendication 1, laquelle composition, dans son état durci final, a une résistance à la compression d'au moins 50 MPa.

10. Composition selon la revendication 1, laquelle composition, dans son état durci final, a une résistance à la traction d'au moins 40 MPa.

11. Composition selon la revendication 1, laquelle composition, dans son état durci final, a un module d'élasticité d'au moins 1500 MPa.

12. Composition selon la revendication 1, laquelle composition, dans son état durci final, a une résistance à la compression d'au moins 50 MPa, une résistance à la traction d'au moins 40 MPa, et un module d'élasticité d'au moins 1500 MPa.
